# EUROPEAN PATENT APPLICATION

(11) **EP 4 763 978 A1**
(43) Date of publication of application: **24.06.2026**
(21) Application number: 25222193.2
(22) Date of filing: 10.12.2025
(51) Int. Cl.: C12N 5/0793

(54) **OTIC PROGENITOR CELL SURFACE MARKERS**

(30) Priority: 13.12.2024 EP 24219778
(71) Applicant: WDI 2 ApS, 2765 Smørum (DK)
(72) Inventor: NAVNTOFT, Charlotte Amalie, DK-2765 Smørum (DK); RAMIREZ, Juan Carlos Vilaescusa, DK-2760 Måløv (DK); AZEVEDO, Carla, DK-2760 Måløv (DK); JENSEN, Simone Møller, DK-2760 Måløv (DK); KANEKO, Kan, DK-2760 Måløv (DK)
(74) Representative: Demant

(57) **Abstract**

The present invention relates to a cell population of spiral ganglion neural progenitors (SGNPs) and methods how to isolate these cells from an in-vitro mixed cell population. The cell population expresses one or more markers, wherein the one or more marker is CXCR4, SUSD2, LPAR3, EPHA5, CHRNA3, REEP1, NPFFR2, SORCS3, NFASC, GABRB3, KCNB2, GRM8, DLL3, and/or KCNH8.

## Description

### Technical field

The present invention relates to a cell population comprising spiral ganglion neural progenitors (SGNPs) and methods how to isolate these cells from an in-vitro mixed cell population. The cell population expresses one or more markers, wherein the one or more marker is CXCR4,SUSD2, LPAR3, EPHA5, CHRNA3, REEP1, NPFFR2, SORCS3, NFASC, GABRB3, KCNB2, GRM8, DLL3, and/or KCNH8.

### Background

Sensorineural hearing loss (SNHL) is a prevalent global sensory disorder often attributed to the loss or dysfunction of mechanosensory hair cells (HCs) and spiral ganglion neurons (SGNs) within the cochlea.

Otic progenitor cells (OPCs) are specialized progenitor cells involved in the development of the inner ear, which houses the sensory organs responsible for hearing and balance, including the cochlea and vestibular system. These cells originate from the otic placode, a thickened region of the ectoderm that gives rise to the otic vesicle, an essential structure in the early stages of inner ear development. Otic progenitor cells are multipotent, meaning they have the capacity to differentiate into various cell types within the inner ear, such as hair cells, supporting cells, and neurons. These differentiated cells play crucial roles in the detection of sound, maintenance of balance, and overall auditory and vestibular function. The ability of OPCs to proliferate, migrate, and respond to developmental cues makes them vital for the proper formation of the complex architecture of the inner ear.

Research into otic progenitor cells has significant implications for regenerative medicine, particularly in developing therapies for hearing loss and balance disorders, which often result from the loss or dysfunction of sensory hair cells and supporting cells in the inner ear. Humans lack the natural capacity to regenerate these critical cells after damage, making OPCs a key focus for potential therapeutic interventions.

Current treatments, such as hearing aids and cochlear implants, do not cure hearing loss but rather help manage it by amplifying sound or directly stimulating the auditory nerve, bypassing the damaged sensory cells. While gene therapy, stem cell therapy, and pharmaceutical approaches are being explored to regenerate or protect OPCs, such as spiral ganglion neural progenitors and spiral ganglion neurons, these strategies are still largely experimental and face numerous hurdles, including the need to precisely identify and target the correct cells, ensure long-term safety, and effectively integrate new cells into the existing auditory circuitry.

### Summary

The present invention relates to an in vitro cell population comprising spiral ganglion neural progenitors (SGNPs) and methods how to isolate these cells from an in-vitro mixed cell population. The present invention relates to an isolated cell population comprising spiral ganglion neural progenitors (SGNPs) and methods how to isolate these cells from an in-vitro mixed cell population.

SGNPs are specifically committed to developing into the neuronal cells of the spiral ganglion. These progenitors are part of the broader pool of otic progenitor cells but are directed towards forming the neurons that transmit auditory information from the cochlear hair cells to the brainstem. Thus, SGNPs have the potential to regenerate or replace the neurons that transmit sound information from the cochlea to the brain. These neurons are essential for hearing, as they form the communication link between sensory hair cells in the inner ear and the auditory nerve, which sends signals to the brain for sound interpretation. Damage to spiral ganglion neurons, often caused by aging, noise exposure, or disease, leads to sensorineural hearing loss, one of the most common forms of hearing impairment.

Specifically, herein are disclosed novel cell surface markers the expression of which is indicative of SGNPs. Current stem cell-based therapy remains largely experimental and faces several challenges, such as the requirement for an enriched and/or a pure cell population and the successful integration of new cells into the existing auditory circuitry. The cell population identified by the marker disclosed here addresses these issues by offering an in-vitro population comprising spiral ganglion neural progenitors (SGNPs) sorted from an otic progenitor cell mix.

A first aspect of the invention relates to an in-vitro cell population comprising spiral ganglion neural progenitors (SGNPs), wherein said population expresses one or more markers, wherein the one or more marker is CXCR4, SUSD2, LPAR3, EPHA5, CHRNA3, REEP1, NPFFR2, SORCS3, NFASC, GABRB3, KCNB2, GRM8, DLL3, and/or KCNH8.

Accordingly, a first aspect of the invention relates to an isolated cell population comprising spiral ganglion neural progenitors (SGNPs), wherein said population expresses one or more markers, wherein the one or more marker is CXCR4, SUSD2, LPAR3, EPHA5, CHRNA3, REEP1, NPFFR2, SORCS3, NFASC, GABRB3, KCNB2, GRM8, DLL3, and/or KCNH8.

A second aspect of the invention relates to a method to identify spiral ganglion neural progenitors (SGNPs) from an in-vitro mixed cell population, the method comprising the step of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population;
thereby identifying a population comprising SGNPs from the in-vitro mixed population.

Accordingly, a second aspect of the invention relates to a method to identify spiral ganglion neural progenitors (SGNPs) from an isolated mixed cell population, the method comprising the step of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population;
thereby identifying a population comprising SGNPs from the isolated mixed population, such as an isolated population comprising SGNPs.

A third aspect of the invention relates to a method to isolate spiral ganglion neural progenitors (SGNPs) from an in-vitro mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
b. Isolating the cells expressing at least one of the markers;
thereby isolating a population of SGNPs from the in-vitro mixed population.

A fourth aspect of the invention relates to a method to enrich spiral ganglion neural progenitors (SGNPs) from an in-vitro mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
b. Isolating the cells expressing at least one of the markers;
thereby enriching a population comprising SGNPs from the in-vitro mixed population.

Accordingly, a fourth aspect of the invention relates to a method to enrich spiral ganglion neural progenitors (SGNPs) from an isolated mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population; and
b. Isolating the cells expressing at least one of the markers;
thereby enriching a population comprising SGNPs from the isolated mixed population.

A fifth aspect of the invention relates to an in-vitro cell population comprising spiral ganglion neural progenitors (SGNPs) obtained by the method comprising following steps:
a. Plating human pluripotent stem cells;
b. Culturing the pluripotent stem cells in medium;
c. Differentiating of the pluripotent stem cells in medium promoting the differentiation to otic progenitor cells;
d. Differentiating the cells of step c) in medium activating BMP signalling;
e. Culturing the cells of step d) in medium comprising a BMP signal inhibitor and bFGF; and
f. Proliferating the cells of step e) to obtain SGNPs;
g. isolating cells from the population of step f) that exhibit one or more marker is CXCR4, SUSD2, LPAR3, EPHA5, CHRNA3, REEP1, NPFFR2, SORCS3, CXCR4, NFASC, GABRB3, KCNB2, GRM8, DLL3, and/or KCNH8.

Accordingly, a fifth aspect of the invention relates to an isolated cell population comprising spiral ganglion neural progenitors (SGNPs) obtained by the method comprising following steps:
a. Plating human pluripotent stem cells;
b. Culturing the pluripotent stem cells in medium;
c. Differentiating of the pluripotent stem cells in medium promoting the differentiation to otic progenitor cells;
d. Differentiating the cells of step c) in medium activating BMP signalling;
e. Culturing the cells of step d) in medium comprising a BMP signal inhibitor and bFGF; and
f. Proliferating the cells of step e) to obtain SGNPs;
g. isolating cells from the population of step f) that exhibit one or more marker is CXCR4, SUSD2, LPAR3, EPHA5, CHRNA3, REEP1, NPFFR2, SORCS3, CXCR4, NFASC, GABRB3, KCNB2, GRM8, DLL3, and/or KCNH8.

A sixth aspect of the invention relates to a kit for isolating the in-vitro population comprising cells comprising spiral ganglion neural progenitors (SGNPs) as described herein, said kit comprising:
a. labelled binding members arranged to bind one or more markers from CXCR4, SUSD2, LPAR3, EPHA5, CHRNA3, REEP1, NPFFR2, SORCS3, NFASC, GABRB3, KCNB2, GRM8, DLL3 and/or KCNH8, preferably wherein said binding members are antibody(ies), nanobodies or microbodies specific for said markers, or magnetic beads conjugated to antibody(ies), nanobodies or microbodies specific for said markers;
b. Instructions performing the method as described herein.

Accordingly, a sixth aspect of the invention relates to a kit for isolating the isolated population comprising spiral ganglion neural progenitors (SGNPs) as described herein, said kit comprising:
a. labelled binding members arranged to bind one or more markers from CXCR4,SUSD2, LPAR3, EPHA5, CHRNA3, REEP1, NPFFR2, SORCS3, NFASC, GABRB3, KCNB2, GRM8, DLL3 and/or KCNH8, preferably wherein said binding members are antibody(ies), nanobodies or microbodies specific for said markers, or magnetic beads conjugated to antibody(ies), nanobodies or microbodies specific for said markers;
b. Instructions performing the method as described herein.

A seventh aspect of the invention relates to a vial comprising cell culture medium and an in-vitro cell population as described herein.

Accordingly, a seventh aspect of the invention relates to a vial comprising cell culture medium and an isolated cell population as described herein.

### Description of Figures

**Figure 1****:** Single-cell RNA sequencing analysis of the cells produced by specific protocol to generate OPCs. UMAP plot displaying the results of unbiased clustering of single OPCs. The numbers refer to identified clusters.
**Figure 2****:** *ISL1, POU4F1* and *NEUROD1* identify cluster 6 as the one containing spiral ganglion neural progenitor cells.
**Figure 3****:** *SUSD2, LPAR3, EPHA5, CHRNA3, REEP1* and *NPFFR2* are mainly expressed in cluster 6.
**Figure 4****:** *SORCS3, CXCR4, NFASC, GABRB3, KCNB2* and *GRM8* are mainly expressed in cluster 6.
**Figure 5****:** *DLL3* and *KCNH8* are mainly expressed in cluster 6. EPCAM can be used to deplete or isolate clusters 9, 10 and 11.
**Figure 6****:** Flow cytometry analyses showing the percentage of single positive cells for CXCR4 (left), and double positive for ISL1+CXCR4+ (middle) and for POU4F1 +CXCR4+ (right).

### Detailed description

### Definitions

Unless defined otherwise, all technical and scientific terms used herein have the same meaning as is commonly understood by one of skill in art to which the subject matter herein belongs. As used herein, the following definitions are supplied to facilitate the understanding of the present invention. The term "comprise" is generally used in the sense of include, that is to say permitting the presence of one or more features or components. In addition, as used in the specification and claims, the language "comprising" can include analogous embodiments described in terms of "consisting of" and/or "consisting essentially of".

As used in the specification and claims, the term "and/or" used in a phrase such as "A and/or B" herein is intended to include "A and B", "A or B", "A", and "B".

As used herein, the singular forms "a", "an" and "the" include plural referents unless the context clearly states otherwise. Thus, for example, reference to "a cell" includes a plurality of such cells. The term "some embodiments" can include one, or more than one embodiment.

As used herein, the term "cell type" refers to a group of cells having a distinct set of morphological, biochemical, and/or functional characteristics that define the cell type. One of skill in the art will recognize that a cell type can be defined with varying levels of specificity. For example, support cells and sensory cells are distinct cell types, which can be distinguished from one another, but differentiated both from otic progenitor cells (OPCs). Typically, cells of different types may be distinguished from one another based on their differential expression of a variety of genes which are referred to in the art as "markers" of a particular cell type or types (e.g., cell types of a particular lineage).

As used herein, the term "undifferentiated cells" refers to cells that are not terminally differentiated. In some embodiments of the present invention, the undifferentiated cells are human induced pluripotent stem cells. In some embodiments of the present invention the undifferentiated cells are progenitor cells. In some embodiments of the present invention, the progenitor cells are spiral ganglion neural progenitors. In some embodiments of the present invention, the undifferentiated cells are progenitor cells or precursor cells of a cell type of interest.

Otic progenitor cells (OPCs) may be defined as early-stage cells in the developing inner ear that have the potential to differentiate into various specialized cell types, such as sensory hair cells, supporting cells, and neurons. These cells can play a key role in auditory and vestibular functions, making them a focus of research for potential therapies targeting hearing loss and inner ear regeneration.

Otic progenitor cells can express specific molecular markers like Pax2 or Sox2, which are associated with early inner ear development. Techniques such as immunohistochemistry and quantitative PCR may be used to detect these markers. Additionally, lineage tracing, where cells are genetically labelled to track their differentiation, can confirm whether they give rise to otic cell types. Gene expression profiling and analysis of the cell's developmental context may further support their identification.

Spiral ganglion neural progenitors (SGNPs) may be defined as early-stage cells that have the potential to differentiate into neurons that form the spiral ganglion, a critical structure in the inner ear. The spiral ganglion contains neurons that transmit sound information from the sensory hair cells in the cochlea to the brain.

To identify spiral ganglion neural progenitors the expression of specific molecular markers such as Sox2, Neurogenin1 (Ngn1), and Brn3a can be used, which are involved in early neural development. Immunohistochemistry and quantitative PCR may be employed to detect these markers in the cell population. Lineage tracing techniques, which track the differentiation of labelled progenitor cells over time, may also help confirm whether these progenitors develop into spiral ganglion neurons. Additionally, single-cell RNA sequencing (scRNA-seq) and gene expression profiling may offer insights into the developmental trajectory of these progenitors, helping to verify their identity based on the expression of neural differentiation genes.

The term "cell population" as used herein may refer to a group of cells that exhibit shared characteristics or a common origin within a biological context. These cells may be identified based on factors such as their type, function, location, or the expression of specific molecular markers. A cell population can be either homogeneous, consisting of cells that are similar in type and function, or heterogeneous, where a variety of cell types with different roles are present.

As used herein, the term "marker" may refer to a specific gene, protein, or molecular characteristic that can be utilized to identify, track, or distinguish particular cell types, biological processes, or physiological conditions. Markers can serve as tools for detecting different stages of cellular differentiation, monitoring cellular responses, or diagnosing specific diseases. These markers may be detected through various methods, including immunohistochemistry, polymerase chain reaction (PCR), flow cytometry or sequencing.

A cell surface marker, as used herein, may refer to a specific protein or molecule located on the outer membrane of a cell that serves to identify the cell type, state, or function. Cell surface markers can be detected using antibodies that specifically bind to these markers. In techniques such as flow cytometry or immunofluorescence, antibodies conjugated with fluorescent dyes or other detectable labels can be used to bind the target markers. Through this method, specific cell populations can be visualized, quantified, and analyzed, enabling detailed study of cellular characteristics and behaviors in various biological contexts.

A "cell type-specific marker" is a gene product or modified version thereof that is expressed at a significantly greater level by one or more cell types than by all or most other cell types and whose expression is characteristic of that cell type. Many cell type specific markers are recognized as such in the art.

As used herein, the term "expression of a marker" may refer to the presence of a specific marker in a cell, where the cell is considered to express the marker if it exhibits detectable levels of said marker. Similarly, "absence of a marker" can refer to a situation where the cell does not exhibit detectable levels of the marker, meaning the cell lacks the expression of that marker according to set detection thresholds.

The threshold can be based on detection of the marker via various methods including immunohistochemistry, PCR, sequencing and FACS.

Unless defined otherwise, all numbers used in this specification and claims to indicate content, concentration, ratio, mass, volume, time, temperature, thickness, technical effect, etc., shall in any case be understood as represented by the term "about" or "approximately" modification. Accordingly, unless indicated to the contrary, the numerical parameters set forth in the following specification and attached claims are approximations. It may vary depending upon the desired properties and effects sought to be obtained by the present disclosure, and each numerical parameter should be interpreted in accordance with the number of significant digits and conventional rounding methods, or as understood by those skilled in the art, to those skilled in the art.

Although the numerical ranges and parameters setting forth the broad scope of the disclosure are approximations, the numerical values set forth in the specific examples are provided as precisely as possible. Any numerical value, however, inherently contain certain errors necessarily resulting from the standard deviation found in their respective testing measurements. Every numerical range given throughout this specification will include every narrower numerical range that falls within such broader numerical range, as if such narrower numerical ranges were all expressly written herein. The term "identity" with respect to a polynucleotide or a polypeptide is defined herein as the percentage of nucleic acids or amino acids, respectively, in the candidate sequence that are identical to the residues of a corresponding native polynucleotide or polypeptide, after aligning the sequences and introducing gaps, if necessary, to achieve the maximum percent identity, and considering any conservative substitutions according to the NCIUB rules (hftp://www.chem.qmul.ac.uk/iubmb/misc/naseq.html; NC-IUB, Eur J Biochem (1985)) as part of the sequence identity. Neither 5' or 3' extensions nor insertions (for nucleic acids) or N' or C' extensions nor insertions (for polypeptides) result in a reduction of identity, similarity or homology. Methods and computer programs for the alignments are well known in the art. Throughout the present disclosure, the term "at least 70%" when referring to a percentage of sequence identity shall refer to a sequence identity of at least 70%, such as at least 71%, such as at least 72%, such as at least 73%, such as at least 74%, such as at least 75%, such as at least 76%, such as at least 77%, such as at least 78%, such as at least 79%, such as at least 80%, such as at least 81%, such as at least 82%, such as at least 83%, such as at least 84%, such as at least 85%, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98%, such as at least 99%, such as 100%.

### SGNP marker

As described herein, the present invention relates to an in-vitro cell population comprising spiral ganglion neural progenitors (SGNPs) characterized by the expression of specific cell surface molecular markers that define their identity and potential for differentiation. Accordingly, the present invention relates to an isolated cell population comprising spiral ganglion neural progenitors (SGNPs) characterized by the expression of specific molecular markers that define their identity and potential for differentiation. SGNPs are a critical cell type for regenerative medicine and auditory research, as they have the ability to give rise to spiral ganglion neurons, which transmit auditory signals from the inner ear to the brain. This cell population may express key neural progenitor markers such as CXCR4, SUSD2, LPAR3, EPHA5, CHRNA3, REEP1, NPFFR2, SORCS3, NFASC, GABRB3, KCNB2, GRM8, DLL3, and/or KCNH8, along with additional markers indicative of their capacity for neuronal differentiation. The invention provides a novel and efficient platform for generating and maintaining SGNPs in vitro, facilitating studies on neural development, hearing restoration therapies, and drug screening for auditory disorders. Thus, the present invention relates to an in-vitro cell population comprising spiral ganglion neural progenitors (SGNPs), wherein said population expresses one or more markers, wherein the one or more marker is CXCR4,SUSD2, LPAR3, EPHA5, CHRNA3, REEP1, NPFFR2, SORCS3, NFASC, GABRB3, KCNB2, GRM8, DLL3, and/or KCNH8.

In some embodiments, the one or more marker is CXCR4, SUSD2, LPAR3, EPHA5, CHRNA3, REEP1, NPFFR2, SORCS3, NFASC, KCNB2, GRM8, DLL3, and/or KCNH8.

As described herein, in some embodiments, the population expresses said one or more markers, i.e the markers are present at the protein level.

However, it will be understood that, for isolation and/or validation purposes, and depending on the selected protocol, the one or more markers may alternatively be detected or assessed at the mRNA level.

Thus, the present invention relates to an isolated cell population comprising spiral ganglion neural progenitors (SGNPs), wherein said population expresses one or more markers, wherein the one or more marker is CXCR4, SUSD2, LPAR3, EPHA5, CHRNA3, REEP1, NPFFR2, SORCS3, NFASC, GABRB3, KCNB2, GRM8, DLL3, and/or KCNH8.

In some embodiments, the one or more marker is CXCR4, SUSD2, LPAR3, EPHA5, CHRNA3, REEP1, NPFFR2, SORCS3, NFASC, KCNB2, GRM8, DLL3, and/or KCNH8.

In some embodiments, the in-vitro cell population expresses one or more markers, wherein the one or more marker is CXCR4, SUSD2, LPAR3, CHRNA3, REEP1, NPFFR2, SORCS3, NFASC, GABRB3, KCNB2, GRM8, DLL3, and/or KCNH8.

In some embodiments, the isolated cell population expresses one or more markers, wherein the one or more marker is CXCR4, SUSD2, LPAR3, CHRNA3, REEP1, NPFFR2, SORCS3, NFASC, GABRB3, KCNB2, GRM8, DLL3, and/or KCNH8.

In some embodiments, said one or more markers are SUSD2, LPAR3, EPHA5, CHRNA3, REEP1, and/or NPFFR2.

In some embodiments, said one or more markers are SUSD2, LPAR3, CHRNA3, REEP1, and/or NPFFR2.

In some embodiments, said one or more markers are CXCR4, SORCS3, NFASC, GABRB3, KCNB2, and/or GRM8.

In some embodiments, said one or more markers are DLL3 and/or KCNH8.

In some embodiments, said one or more markers is CXCR4.

In some embodiments, said population does not express EPCAM.

SUSD2 (Sushi Domain Containing 2) is a transmembrane protein that contains a sushi domain, which may be involved in cell adhesion, immune response, and potentially cancer progression. In some embodiments, SUSD2 comprises the sequence having the accession number Q9UGT4 in the Uniprot database or a sequence encoding a homologue, such as an ortholog of said SUSD2 sharing at least 70%, such as at least 80%, for example at least 90%, such as at least 95% sequence identity therewith.

LPAR3 (Lysophosphatidic Acid Receptor 3) is a member of the G protein-coupled receptor family that binds to lysophosphatidic acid (LPA), a lipid signalling molecule. This receptor may be involved in processes such as cell proliferation, migration, and survival. In some embodiments, LPAR3 comprises the sequence having the accession number Q9UBY5 in the Uniprot database or a sequence encoding a homologue, such as an ortholog of said LPAR3 sharing at least 70%, such as at least 80%, for example at least 90%, such as at least 95% sequence identity therewith. EPHA5 (Ephrin Type-A Receptor 5) is part of the ephrin receptor family, which is involved in cell signalling related to neural development and synaptic plasticity. In some embodiments, EPHA5 comprises the sequence having the accession number P54756 in the Uniprot database or a sequence encoding a homologue, such as an ortholog of said EPHA5 sharing at least 70%, such as at least 80%, for example at least 90%, such as at least 95% sequence identity therewith. CHRNA3 (Cholinergic Receptor Nicotinic Alpha 3 Subunit) encodes a subunit of the nicotinic acetylcholine receptor, which can be involved in neurotransmission. This receptor can be found in the nervous system, and its activation plays a role in processes such as synaptic transmission, learning, and memory. In some embodiments, CHRNA3 comprises the sequence having the accession number P32297 in the Uniprot database or a sequence encoding a homologue, such as an ortholog of said CHRNA3 sharing at least 70%, such as at least 80%, for example at least 90%, such as at least 95% sequence identity therewith.

REEP1 (Receptor Expression-Enhancing Protein 1) can be involved in enhancing the cell surface expression of odorant receptors and other G protein-coupled receptors (GPCRs). In some embodiments, REEP1 comprises the sequence having the accession number Q9H902 in the Uniprot database or a sequence encoding a homologue, such as an ortholog of said REEP1 sharing at least 70%, such as at least 80%, for example at least 90%, such as at least 95% sequence identity therewith.

NPFFR2 (Neuropeptide FF Receptor 2): NPFFR2 is a receptor for neuropeptides FF and AF, which can be involved in modulating pain, opioid tolerance, and energy homeostasis. In some embodiments, NPFFR2 comprises the sequence having the accession number Q9Y5X5 in the Uniprot database or a sequence encoding a homologue, such as an ortholog of said NPFFR2 sharing at least 70%, such as at least 80%, for example at least 90%, such as at least 95% sequence identity therewith.

SORCS3 (Sortilin-Related VPS10 Domain Containing Receptor 3) is a receptor that belongs to the VPS10P domain receptor family, which can be involved in intracellular protein trafficking and signalling. In some embodiments, SORCS3 comprises the sequence having the accession number Q9UPU3in the Uniprot database or a sequence encoding a homologue, such as an ortholog of said SORCS3 sharing at least 70%, such as at least 80%, for example at least 90%, such as at least 95% sequence identity therewith.

CXCR4 (C-X-C Chemokine Receptor Type 4) is a chemokine receptor that can play a role in immune cell trafficking and the homing of hematopoietic stem cells. In some embodiments, CXCR4 comprises the sequence having the accession number P61073 in the Uniprot database or a sequence encoding a homologue, such as an ortholog of said CXCR4 sharing at least 70%, such as at least 80%, for example at least 90%, such as at least 95% sequence identity therewith. NFASC (Neurofascin) encodes a protein involved in the organization of nodes of Ranvier and the paranodal regions of myelinated axons. In some embodiments, NFASC comprises the sequence having the accession number O94856 in the Uniprot database or a sequence encoding a homologue, such as an ortholog of said NFASC sharing at least 70%, such as at least 80%, for example at least 90%, such as at least 95% sequence identity therewith.

GABRB3 (Gamma-Aminobutyric Acid Type A Receptor Subunit Beta3) encodes a subunit of the GABA-A receptor, which can be an inhibitory neurotransmitter receptor in the brain. In some embodiments, GABRB3 comprises the sequence having the accession number P28472 in the Uniprot database or a sequence encoding a homologue, such as an ortholog of said GABRB3 sharing at least 70%, such as at least 80%, for example at least 90%, such as at least 95% sequence identity therewith.

KCNB2 (Potassium Voltage-Gated Channel Subfamily B Member 2) encodes a voltage-gated potassium channel subunit, which can be important for regulating electrical signalling in neurons. In some embodiments, KCNB2 comprises the sequence having the accession number Q92953 in the Uniprot database or a sequence encoding a homologue such as an ortholog of said KCNB2 sharing at least 70%, such as at least 80%, for example at least 90%, such as at least 95% sequence identity therewith.

GRM8 (Glutamate Metabotropic Receptor 8) encodes a metabotropic glutamate receptor, which can be involved in modulating synaptic transmission and plasticity in the central nervous system. In some embodiments, GRM8 comprises the sequence having the accession number O00222 in the Uniprot database or a sequence encoding a homologue, such as an ortholog of said GRM8 sharing at least 70%, such as at least 80%, for example at least 90%, such as at least 95% sequence identity therewith.

DLL3 (Delta-Like 3) is a member of the Notch signalling pathway, which can be involved in cell differentiation and development. In some embodiments, DLL3 comprises the sequence having the accession number Q9NYJ7 in the Uniprot database or a sequence encoding a homologue, such as an ortholog of said DLL3 sharing at least 70%, such as at least 80%, for example at least 90%, such as at least 95% sequence identity therewith.

KCNH8 (Potassium Voltage-Gated Channel Subfamily H Member 8) encodes a potassium voltage-gated channel that can play a role in regulating neuronal excitability. In some embodiments, KCNH8 comprises the sequence having the accession number Q96L42 in the Uniprot database or a sequence encoding a homologue, such as an ortholog of said KCNH8 sharing at least 70%, such as at least 80%, for example at least 90%, such as at least 95% sequence identity therewith. EPCAM (Epithelial Cell Adhesion Molecule), as used herein, may refer to a cell surface glycoprotein involved in cell adhesion, proliferation, differentiation, and migration. In some embodiments, EPCAM comprises the sequence having the accession number P16422 in the Uniprot database or a sequence encoding a homologue, such as an ortholog of said EPCAM sharing at least 70%, such as at least 80%, for example at least 90%, such as at least 95% sequence identity therewith.

In some embodiments,
a. the marker CXCR4 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
b. the marker SUSD2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
c. the marker LPAR3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
d. the marker EPHA5 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
e. the marker CHRNA3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
f. the marker REEP1 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
g. the marker NPFFR2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
h. the marker SORCS3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
i. the marker NFASC is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
j. the marker GABRB3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
k. the marker KCNB2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
l. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
m. the marker KCNH8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population; and/or
n. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population.

In some embodiments, the marker EPCAM is expressed in at most 99%, such as at most 90% such as at most 75%, such as at most 50%, such as at most 25%, such as at most 20%, such as at most 15%, such as at most 10%, such as at most 7.5%, such as at most 5%, such as at most 4%, such as at most 3%, such as at most 2%, such as at most 1%, such as 0% of the cells of the population.

In some embodiments, said population further expresses ISL1, POU4F1, and/or NEUROD1.

ISL1 (ISL LIM Homeobox 1), as used herein, may refer to a transcription factor that plays a critical role in the development of motor neurons, pancreatic beta cells, and cardiac tissue. ISL1 is part of the LIM-homeodomain family of transcription factors, which are involved in regulating gene expression during embryonic development. In some embodiments, ISL1 comprises the sequence having the accession number P61371 in the Uniprot database or a sequence encoding a homologue, such as an ortholog of said ISL1 sharing at least 70%, such as at least 80%, for example at least 90%, such as at least 95% sequence identity therewith.

POU4F1 (POU Class 4 Homeobox 1), as used herein, may refer to a transcription factor that is primarily involved in the regulation of gene expression in sensory neurons, particularly in the development and maintenance of retinal ganglion cells and other types of sensory neurons. It belongs to the POU-domain family of transcription factors, which are known for their roles in controlling the expression of genes related to neural differentiation and development. In some embodiments, POU4F1 comprises the sequence having the accession number Q01851 in the Uniprot database or a sequence encoding a homologue, such as an ortholog of said POU4F1 sharing at least 70%, such as at least 80%, for example at least 90%, such as at least 95% sequence identity therewith.

NEUROD1 (Neurogenic Differentiation 1), as used herein, may refer to a basic helix-loop-helix (bHLH) transcription factor that plays a key role in the development and differentiation of neurons and endocrine cells. In some embodiments, NEUROD1 comprises the sequence having the accession number Q13562 in the Uniprot database or a sequence encoding a homologue, such as an ortholog of said NEUROD1 sharing at least 70%, such as at least 80%, for example at least 90%, such as at least 95% sequence identity therewith.

In some embodiments, said one or more markers are CXCR4; SUSD2; LPAR3; EPHA5; CHRNA3; REEP1; NPFFR2; SORCS3; NFASC; GABRB3; KCNB2; GRM8; DLL3; and KCNH8, optionally wherein said isolated population does not express EPCAM.

In some embodiments, said one or more markers are CXCR4; SUSD2; LPAR3; EPHA5; CHRNA3; REEP1; NPFFR2; SORCS3; NFASC; KCNB2; GRM8; DLL3; and KCNH8, optionally wherein said isolated population does not express EPCAM.

In some embodiments, said one or more markers are CXCR4, optionally wherein said isolated population does not express EPCAM.

In some embodiments, said one or more markers are SUSD2, optionally wherein said isolated population does not express EPCAM.

In some embodiments, said one or more markers are LPAR3, optionally wherein said isolated population does not express EPCAM.

In some embodiments, said one or more markers are EPHA5, optionally wherein said isolated population does not express EPCAM.

In some embodiments, said one or more markers are CHRNA3, optionally wherein said isolated population does not express EPCAM.

In some embodiments, said one or more markers are REEP1, optionally wherein said isolated population does not express EPCAM.

In some embodiments, said one or more markers are NPFFR2, optionally wherein said isolated population does not express EPCAM.

In some embodiments, said one or more markers are SORCS3, optionally wherein said isolated population does not express EPCAM.

In some embodiments, said one or more markers are NFASC, optionally wherein said isolated population does not express EPCAM.

In some embodiments, said one or more markers are GABRB3, optionally wherein said isolated population does not express EPCAM.

In some embodiments, said one or more markers are KCNB2, optionally wherein said isolated population does not express EPCAM.

In some embodiments, said one or more markers are GRM8, optionally wherein said isolated population does not express EPCAM.

In some embodiments, said one or more markers are DLL3, optionally wherein said isolated population does not express EPCAM.

In some embodiments, said one or more markers are KCNH8, optionally wherein said isolated population does not express EPCAM.

In some embodiments, said one or more markers are CXCR4 and SUSD2, optionally wherein said isolated population does not express EPCAM.

In some embodiments, said one or more markers are CXCR4 and LPAR3, optionally wherein said isolated population does not express EPCAM.

In some embodiments, said one or more markers are CXCR4 and EPHA5, optionally wherein said isolated population does not express EPCAM.

In some embodiments, said one or more markers are CXCR4 and CHRNA3, optionally wherein said isolated population does not express EPCAM.

In some embodiments, said one or more markers are CXCR4 and REEP1, optionally wherein said isolated population does not express EPCAM.

In some embodiments, said one or more markers are CXCR4 and NPFFR2, optionally wherein said isolated population does not express EPCAM.

In some embodiments, said one or more markers are CXCR4 and SORCS3, optionally wherein said isolated population does not express EPCAM.

In some embodiments, said one or more markers are CXCR4 and NFASC, optionally wherein said isolated population does not express EPCAM.

In some embodiments, said one or more markers are CXCR4 and GABRB3, optionally wherein said isolated population does not express EPCAM.

In some embodiments, said one or more markers are CXCR4 and KCNB2, optionally wherein said isolated population does not express EPCAM.

In some embodiments, said one or more markers are CXCR4 and GRM8, optionally wherein said isolated population does not express EPCAM.

In some embodiments, said one or more markers are CXCR4 and DLL3, optionally wherein said isolated population does not express EPCAM.

In some embodiments, said one or more markers are CXCR4 and KCNH8, optionally wherein said isolated population does not express EPCAM.

In some embodiments, said one or more markers are SUSD2 and LPAR3, optionally wherein said isolated population does not express EPCAM.

In some embodiments, said one or more markers are SUSD2 and EPHA5, optionally wherein said isolated population does not express EPCAM.

In some embodiments, said one or more markers are SUSD2 and CHRNA3, optionally wherein said isolated population does not express EPCAM.

In some embodiments, said one or more markers are SUSD2 and REEP1, optionally wherein said isolated population does not express EPCAM.

In some embodiments, said one or more markers are SUSD2 and NPFFR2, optionally wherein said isolated population does not express EPCAM.

In some embodiments, said one or more markers are SUSD2 and SORCS3, optionally wherein said isolated population does not express EPCAM.

In some embodiments, said one or more markers are SUSD2 and NFASC, optionally wherein said isolated population does not express EPCAM.

In some embodiments, said one or more markers are SUSD2 and GABRB3, optionally wherein said isolated population does not express EPCAM.

In some embodiments, said one or more markers are SUSD2 and KCNB2, optionally wherein said isolated population does not express EPCAM.

In some embodiments, said one or more markers are SUSD2 and GRM8, optionally wherein said isolated population does not express EPCAM.

In some embodiments, said one or more markers are SUSD2 and DLL3, optionally wherein said isolated population does not express EPCAM.

In some embodiments, said one or more markers are SUSD2 and KCNH8, optionally wherein said isolated population does not express EPCAM.

In some embodiments, said one or more markers are LPAR3 and EPHA5, optionally wherein said isolated population does not express EPCAM.

In some embodiments, said one or more markers are LPAR3 and CHRNA3, optionally wherein said isolated population does not express EPCAM.

In some embodiments, said one or more markers are LPAR3 and REEP1, optionally wherein said isolated population does not express EPCAM.

In some embodiments, said one or more markers are LPAR3 and NPFFR2, optionally wherein said isolated population does not express EPCAM.

In some embodiments, said one or more markers are LPAR3 and SORCS3, optionally wherein said isolated population does not express EPCAM.

In some embodiments, said one or more markers are LPAR3 and NFASC, optionally wherein said isolated population does not express EPCAM.

In some embodiments, said one or more markers are LPAR3 and GABRB3, optionally wherein said isolated population does not express EPCAM.

In some embodiments, said one or more markers are LPAR3 and KCNB2, optionally wherein said isolated population does not express EPCAM.

In some embodiments, said one or more markers are LPAR3 and GRM8, optionally wherein said isolated population does not express EPCAM.

In some embodiments, said one or more markers are LPAR3 and DLL3, optionally wherein said isolated population does not express EPCAM.

In some embodiments, said one or more markers are LPAR3 and KCNH8, optionally wherein said isolated population does not express EPCAM.

In some embodiments, said one or more markers are EPHA5 and CHRNA3, optionally wherein said isolated population does not express EPCAM.

In some embodiments, said one or more markers are EPHA5 and REEP1, optionally wherein said isolated population does not express EPCAM.

In some embodiments, said one or more markers are EPHA5 and NPFFR2, optionally wherein said isolated population does not express EPCAM.

In some embodiments, said one or more markers are EPHA5 and SORCS3, optionally wherein said isolated population does not express EPCAM.

In some embodiments, said one or more markers are EPHA5 and NFASC, optionally wherein said isolated population does not express EPCAM.

In some embodiments, said one or more markers are EPHA5 and GABRB3, optionally wherein said isolated population does not express EPCAM.

In some embodiments, said one or more markers are EPHA5 and KCNB2, optionally wherein said isolated population does not express EPCAM.

In some embodiments, said one or more markers are EPHA5 and GRM8, optionally wherein said isolated population does not express EPCAM.

In some embodiments, said one or more markers are EPHA5 and DLL3, optionally wherein said isolated population does not express EPCAM.

In some embodiments, said one or more markers are EPHA5 and KCNH8, optionally wherein said isolated population does not express EPCAM.

In some embodiments, said one or more markers are CHRNA3 and REEP1, optionally wherein said isolated population does not express EPCAM.

In some embodiments, said one or more markers are CHRNA3 and NPFFR2, optionally wherein said isolated population does not express EPCAM.

In some embodiments, said one or more markers are CHRNA3 and SORCS3, optionally wherein said isolated population does not express EPCAM.

In some embodiments, said one or more markers are CHRNA3 and NFASC, optionally wherein said isolated population does not express EPCAM.

In some embodiments, said one or more markers are CHRNA3 and GABRB3, optionally wherein said isolated population does not express EPCAM.

In some embodiments, said one or more markers are CHRNA3 and KCNB2, optionally wherein said isolated population does not express EPCAM.

In some embodiments, said one or more markers are CHRNA3 and GRM8, optionally wherein said isolated population does not express EPCAM.

In some embodiments, said one or more markers are CHRNA3 and DLL3, optionally wherein said isolated population does not express EPCAM.

In some embodiments, said one or more markers are CHRNA3 and KCNH8, optionally wherein said isolated population does not express EPCAM.

In some embodiments, said one or more markers are REEP1 and NPFFR2, optionally wherein said isolated population does not express EPCAM.

In some embodiments, said one or more markers are REEP1 and SORCS3, optionally wherein said isolated population does not express EPCAM.

In some embodiments, said one or more markers are REEP1 and NFASC, optionally wherein said isolated population does not express EPCAM.

In some embodiments, said one or more markers are REEP1 and GABRB3, optionally wherein said isolated population does not express EPCAM.

In some embodiments, said one or more markers are REEP1 and KCNB2, optionally wherein said isolated population does not express EPCAM.

In some embodiments, said one or more markers are REEP1 and GRM8, optionally wherein said isolated population does not express EPCAM.

In some embodiments, said one or more markers are REEP1 and DLL3, optionally wherein said isolated population does not express EPCAM.

In some embodiments, said one or more markers are REEP1 and KCNH8, optionally wherein said isolated population does not express EPCAM.

In some embodiments, said one or more markers are NPFFR2 and SORCS3, optionally wherein said isolated population does not express EPCAM.

In some embodiments, said one or more markers are NPFFR2 and NFASC, optionally wherein said isolated population does not express EPCAM.

In some embodiments, said one or more markers are NPFFR2 and GABRB3, optionally wherein said isolated population does not express EPCAM.

In some embodiments, said one or more markers are NPFFR2 and KCNB2, optionally wherein said isolated population does not express EPCAM.

In some embodiments, said one or more markers are NPFFR2 and GRM8, optionally wherein said isolated population does not express EPCAM.

In some embodiments, said one or more markers are NPFFR2 and DLL3, optionally wherein said isolated population does not express EPCAM.

In some embodiments, said one or more markers are NPFFR2 and KCNH8, optionally wherein said isolated population does not express EPCAM.

In some embodiments, said one or more markers are SORCS3 and NFASC, optionally wherein said isolated population does not express EPCAM.

In some embodiments, said one or more markers are SORCS3 and GABRB3, optionally wherein said isolated population does not express EPCAM.

In some embodiments, said one or more markers are SORCS3 and KCNB2, optionally wherein said isolated population does not express EPCAM.

In some embodiments, said one or more markers are SORCS3 and GRM8, optionally wherein said isolated population does not express EPCAM.

In some embodiments, said one or more markers are SORCS3 and DLL3, optionally wherein said isolated population does not express EPCAM.

In some embodiments, said one or more markers are SORCS3 and KCNH8, optionally wherein said isolated population does not express EPCAM.

In some embodiments, said one or more markers are NFASC and GABRB3, optionally wherein said isolated population does not express EPCAM.

In some embodiments, said one or more markers are NFASC and KCNB2, optionally wherein said isolated population does not express EPCAM.

In some embodiments, said one or more markers are NFASC and GRM8, optionally wherein said isolated population does not express EPCAM.

In some embodiments, said one or more markers are NFASC and DLL3, optionally wherein said isolated population does not express EPCAM.

In some embodiments, said one or more markers are NFASC and KCNH8, optionally wherein said isolated population does not express EPCAM.

In some embodiments, said one or more markers are GABRB3 and KCNB2, optionally wherein said isolated population does not express EPCAM.

In some embodiments, said one or more markers are GABRB3 and GRM8, optionally wherein said isolated population does not express EPCAM.

In some embodiments, said one or more markers are GABRB3 and DLL3, optionally wherein said isolated population does not express EPCAM.

In some embodiments, said one or more markers are GABRB3 and KCNH8, optionally wherein said isolated population does not express EPCAM.

In some embodiments, said one or more markers are KCNB2 and GRM8, optionally wherein said isolated population does not express EPCAM.

In some embodiments, said one or more markers are KCNB2 and DLL3, optionally wherein said isolated population does not express EPCAM.

In some embodiments, said one or more markers are KCNB2 and KCNH8, optionally wherein said isolated population does not express EPCAM.

In some embodiments, said one or more markers are GRM8 and DLL3, optionally wherein said isolated population does not express EPCAM.

In some embodiments, said one or more markers are GRM8 and KCNH8, optionally wherein said isolated population does not express EPCAM.

In some embodiments, said one or more markers are DLL3 and KCNH8, optionally wherein said isolated population does not express EPCAM.

In some embodiments, said one or more markers are CXCR4; SUSD2; and LPAR3, optionally wherein said isolated population does not express EPCAM.

In some embodiments, said one or more markers are CXCR4; SUSD2; and EPHA5, optionally wherein said isolated population does not express EPCAM.

In some embodiments, said one or more markers are CXCR4; SUSD2; and CHRNA3, optionally wherein said isolated population does not express EPCAM.

In some embodiments, said one or more markers are CXCR4; SUSD2; and REEP1, optionally wherein said isolated population does not express EPCAM.

In some embodiments, said one or more markers are CXCR4; SUSD2; and NPFFR2, optionally wherein said isolated population does not express EPCAM.

In some embodiments, said one or more markers are CXCR4; SUSD2; and SORCS3, optionally wherein said isolated population does not express EPCAM.

In some embodiments, said one or more markers are CXCR4; SUSD2; and NFASC, optionally wherein said isolated population does not express EPCAM.

In some embodiments, said one or more markers are CXCR4; SUSD2; and GABRB3, optionally wherein said isolated population does not express EPCAM.

In some embodiments, said one or more markers are CXCR4; SUSD2; and KCNB2, optionally wherein said isolated population does not express EPCAM.

In some embodiments, said one or more markers are CXCR4; SUSD2; and GRM8, optionally wherein said isolated population does not express EPCAM.

In some embodiments, said one or more markers are CXCR4; SUSD2; and DLL3, optionally wherein said isolated population does not express EPCAM.

In some embodiments, said one or more markers are CXCR4; SUSD2; and KCNH8, optionally wherein said isolated population does not express EPCAM.

In some embodiments, said one or more markers are CXCR4; LPAR3; and EPHA5, optionally wherein said isolated population does not express EPCAM.

In some embodiments, said one or more markers are CXCR4; LPAR3; and CHRNA3, optionally wherein said isolated population does not express EPCAM.

In some embodiments, said one or more markers are CXCR4; LPAR3; and REEP1, optionally wherein said isolated population does not express EPCAM.

In some embodiments, said one or more markers are CXCR4; LPAR3; and NPFFR2, optionally wherein said isolated population does not express EPCAM.

In some embodiments, said one or more markers are CXCR4; LPAR3; and SORCS3, optionally wherein said isolated population does not express EPCAM.

In some embodiments, said one or more markers are CXCR4; LPAR3; and NFASC, optionally wherein said isolated population does not express EPCAM.

In some embodiments, said one or more markers are CXCR4; LPAR3; and GABRB3, optionally wherein said isolated population does not express EPCAM.

In some embodiments, said one or more markers are CXCR4; LPAR3; and KCNB2, optionally wherein said isolated population does not express EPCAM.

In some embodiments, said one or more markers are CXCR4; LPAR3; and GRM8, optionally wherein said isolated population does not express EPCAM.

In some embodiments, said one or more markers are CXCR4; LPAR3; and DLL3, optionally wherein said isolated population does not express EPCAM.

In some embodiments, said one or more markers are CXCR4; LPAR3; and KCNH8, optionally wherein said isolated population does not express EPCAM.

In some embodiments, said one or more markers are CXCR4; EPHA5; and CHRNA3, optionally wherein said isolated population does not express EPCAM.

In some embodiments, said one or more markers are CXCR4; EPHA5; and REEP1, optionally wherein said isolated population does not express EPCAM.

In some embodiments, said one or more markers are CXCR4; EPHA5; and NPFFR2, optionally wherein said isolated population does not express EPCAM.

In some embodiments, said one or more markers are CXCR4; EPHA5; and SORCS3, optionally wherein said isolated population does not express EPCAM.

In some embodiments, said one or more markers are CXCR4; EPHA5; and NFASC, optionally wherein said isolated population does not express EPCAM.

In some embodiments, said one or more markers are CXCR4; EPHA5; and GABRB3, optionally wherein said isolated population does not express EPCAM.

In some embodiments, said one or more markers are CXCR4; EPHA5; and KCNB2, optionally wherein said isolated population does not express EPCAM.

In some embodiments, said one or more markers are CXCR4; EPHA5; and GRM8, optionally wherein said isolated population does not express EPCAM.

In some embodiments, said one or more markers are CXCR4; EPHA5; and DLL3, optionally wherein said isolated population does not express EPCAM.

In some embodiments, said one or more markers are CXCR4; EPHA5; and KCNH8, optionally wherein said isolated population does not express EPCAM.

In some embodiments, said one or more markers are CXCR4; CHRNA3; and REEP1, optionally wherein said isolated population does not express EPCAM.

In some embodiments, said one or more markers are CXCR4; CHRNA3; and NPFFR2, optionally wherein said isolated population does not express EPCAM.

In some embodiments, said one or more markers are CXCR4; CHRNA3; and SORCS3, optionally wherein said isolated population does not express EPCAM.

In some embodiments, said one or more markers are CXCR4; CHRNA3; and NFASC, optionally wherein said isolated population does not express EPCAM.

In some embodiments, said one or more markers are CXCR4; CHRNA3; and GABRB3, optionally wherein said isolated population does not express EPCAM.

In some embodiments, said one or more markers are CXCR4; CHRNA3; and KCNB2, optionally wherein said isolated population does not express EPCAM.

In some embodiments, said one or more markers are CXCR4; CHRNA3; and GRM8, optionally wherein said isolated population does not express EPCAM.

In some embodiments, said one or more markers are CXCR4; CHRNA3; and DLL3, optionally wherein said isolated population does not express EPCAM.

In some embodiments, said one or more markers are CXCR4; CHRNA3; and KCNH8, optionally wherein said isolated population does not express EPCAM.

In some embodiments, said one or more markers are CXCR4; REEP1; and NPFFR2, optionally wherein said isolated population does not express EPCAM.

In some embodiments, said one or more markers are CXCR4; REEP1; and SORCS3, optionally wherein said isolated population does not express EPCAM.

In some embodiments, said one or more markers are CXCR4; REEP1; and NFASC, optionally wherein said isolated population does not express EPCAM.

In some embodiments, said one or more markers are CXCR4; REEP1; and GABRB3, optionally wherein said isolated population does not express EPCAM.

In some embodiments, said one or more markers are CXCR4; REEP1; and KCNB2, optionally wherein said isolated population does not express EPCAM.

In some embodiments, said one or more markers are CXCR4; REEP1; and GRM8, optionally wherein said isolated population does not express EPCAM.

In some embodiments, said one or more markers are CXCR4; REEP1; and DLL3, optionally wherein said isolated population does not express EPCAM.

In some embodiments, said one or more markers are CXCR4; REEP1; and KCNH8, optionally wherein said isolated population does not express EPCAM.

In some embodiments, said one or more markers are CXCR4; NPFFR2; and SORCS3, optionally wherein said isolated population does not express EPCAM.

In some embodiments, said one or more markers are CXCR4; NPFFR2; and NFASC, optionally wherein said isolated population does not express EPCAM.

In some embodiments, said one or more markers are CXCR4; NPFFR2; and GABRB3, optionally wherein said isolated population does not express EPCAM.

In some embodiments, said one or more markers are CXCR4; NPFFR2; and KCNB2, optionally wherein said isolated population does not express EPCAM.

In some embodiments, said one or more markers are CXCR4; NPFFR2; and GRM8, optionally wherein said isolated population does not express EPCAM.

In some embodiments, said one or more markers are CXCR4; NPFFR2; and DLL3, optionally wherein said isolated population does not express EPCAM.

In some embodiments, said one or more markers are CXCR4; NPFFR2; and KCNH8, optionally wherein said isolated population does not express EPCAM.

In some embodiments, said one or more markers are CXCR4; SORCS3; and NFASC, optionally wherein said isolated population does not express EPCAM.

In some embodiments, said one or more markers are CXCR4; SORCS3; and GABRB3, optionally wherein said isolated population does not express EPCAM.

In some embodiments, said one or more markers are CXCR4; SORCS3; and KCNB2, optionally wherein said isolated population does not express EPCAM.

In some embodiments, said one or more markers are CXCR4; SORCS3; and GRM8, optionally wherein said isolated population does not express EPCAM.

In some embodiments, said one or more markers are CXCR4; SORCS3; and DLL3, optionally wherein said isolated population does not express EPCAM.

In some embodiments, said one or more markers are CXCR4; SORCS3; and KCNH8, optionally wherein said isolated population does not express EPCAM.

In some embodiments, said one or more markers are CXCR4; NFASC; and GABRB3, optionally wherein said isolated population does not express EPCAM.

In some embodiments, said one or more markers are CXCR4; NFASC; and KCNB2, optionally wherein said isolated population does not express EPCAM.

In some embodiments, said one or more markers are CXCR4; NFASC; and GRM8, optionally wherein said isolated population does not express EPCAM.

In some embodiments, said one or more markers are CXCR4; NFASC; and DLL3, optionally wherein said isolated population does not express EPCAM.

In some embodiments, said one or more markers are CXCR4; NFASC; and KCNH8, optionally wherein said isolated population does not express EPCAM.

In some embodiments, said one or more markers are CXCR4; GABRB3; and KCNB2, optionally wherein said isolated population does not express EPCAM.

In some embodiments, said one or more markers are CXCR4; GABRB3; and GRM8, optionally wherein said isolated population does not express EPCAM.

In some embodiments, said one or more markers are CXCR4; GABRB3; and DLL3, optionally wherein said isolated population does not express EPCAM.

In some embodiments, said one or more markers are CXCR4; GABRB3; and KCNH8, optionally wherein said isolated population does not express EPCAM.

In some embodiments, said one or more markers are CXCR4; KCNB2; and GRM8, optionally wherein said isolated population does not express EPCAM.

In some embodiments, said one or more markers are CXCR4; KCNB2; and DLL3, optionally wherein said isolated population does not express EPCAM.

In some embodiments, said one or more markers are CXCR4; KCNB2; and KCNH8, optionally wherein said isolated population does not express EPCAM.

In some embodiments, said one or more markers are CXCR4; GRM8; and DLL3, optionally wherein said isolated population does not express EPCAM.

In some embodiments, said one or more markers are CXCR4; GRM8; and KCNH8, optionally wherein said isolated population does not express EPCAM.

In some embodiments, said one or more markers are CXCR4; DLL3; and KCNH8, optionally wherein said isolated population does not express EPCAM.

In some embodiments, said one or more markers are SUSD2; LPAR3; and EPHA5, optionally wherein said isolated population does not express EPCAM.

In some embodiments, said one or more markers are SUSD2; LPAR3; and CHRNA3, optionally wherein said isolated population does not express EPCAM.

In some embodiments, said one or more markers are SUSD2; LPAR3; and REEP1, optionally wherein said isolated population does not express EPCAM.

In some embodiments, said one or more markers are SUSD2; LPAR3; and NPFFR2, optionally wherein said isolated population does not express EPCAM.

In some embodiments, said one or more markers are SUSD2; LPAR3; and SORCS3, optionally wherein said isolated population does not express EPCAM.

In some embodiments, said one or more markers are SUSD2; LPAR3; and NFASC, optionally wherein said isolated population does not express EPCAM.

In some embodiments, said one or more markers are SUSD2; LPAR3; and GABRB3, optionally wherein said isolated population does not express EPCAM.

In some embodiments, said one or more markers are SUSD2; LPAR3; and KCNB2, optionally wherein said isolated population does not express EPCAM.

In some embodiments, said one or more markers are SUSD2; LPAR3; and GRM8, optionally wherein said isolated population does not express EPCAM.

In some embodiments, said one or more markers are SUSD2; LPAR3; and DLL3, optionally wherein said isolated population does not express EPCAM.

In some embodiments, said one or more markers are SUSD2; LPAR3; and KCNH8, optionally wherein said isolated population does not express EPCAM.

In some embodiments, said one or more markers are SUSD2; EPHA5; and CHRNA3, optionally wherein said isolated population does not express EPCAM.

In some embodiments, said one or more markers are SUSD2; EPHA5; and REEP1, optionally wherein said isolated population does not express EPCAM.

In some embodiments, said one or more markers are SUSD2; EPHA5; and NPFFR2, optionally wherein said isolated population does not express EPCAM.

In some embodiments, said one or more markers are SUSD2; EPHA5; and SORCS3, optionally wherein said isolated population does not express EPCAM.

In some embodiments, said one or more markers are SUSD2; EPHA5; and NFASC, optionally wherein said isolated population does not express EPCAM.

In some embodiments, said one or more markers are SUSD2; EPHA5; and GABRB3, optionally wherein said isolated population does not express EPCAM.

In some embodiments, said one or more markers are SUSD2; EPHA5; and KCNB2, optionally wherein said isolated population does not express EPCAM.

In some embodiments, said one or more markers are SUSD2; EPHA5; and GRM8, optionally wherein said isolated population does not express EPCAM.

In some embodiments, said one or more markers are SUSD2; EPHA5; and DLL3, optionally wherein said isolated population does not express EPCAM.

In some embodiments, said one or more markers are SUSD2; EPHA5; and KCNH8, optionally wherein said isolated population does not express EPCAM.

In some embodiments, said one or more markers are SUSD2; CHRNA3; and REEP1, optionally wherein said isolated population does not express EPCAM.

In some embodiments, said one or more markers are SUSD2; CHRNA3; and NPFFR2, optionally wherein said isolated population does not express EPCAM.

In some embodiments, said one or more markers are SUSD2; CHRNA3; and SORCS3, optionally wherein said isolated population does not express EPCAM.

In some embodiments, said one or more markers are SUSD2; CHRNA3; and NFASC, optionally wherein said isolated population does not express EPCAM.

In some embodiments, said one or more markers are SUSD2; CHRNA3; and GABRB3, optionally wherein said isolated population does not express EPCAM.

In some embodiments, said one or more markers are SUSD2; CHRNA3; and KCNB2, optionally wherein said isolated population does not express EPCAM.

In some embodiments, said one or more markers are SUSD2; CHRNA3; and GRM8, optionally wherein said isolated population does not express EPCAM.

In some embodiments, said one or more markers are SUSD2; CHRNA3; and DLL3, optionally wherein said isolated population does not express EPCAM.

In some embodiments, said one or more markers are SUSD2; CHRNA3; and KCNH8, optionally wherein said isolated population does not express EPCAM.

In some embodiments, said one or more markers are SUSD2; REEP1; and NPFFR2, optionally wherein said isolated population does not express EPCAM.

In some embodiments, said one or more markers are SUSD2; REEP1; and SORCS3, optionally wherein said isolated population does not express EPCAM.

In some embodiments, said one or more markers are SUSD2; REEP1; and NFASC, optionally wherein said isolated population does not express EPCAM.

In some embodiments, said one or more markers are SUSD2; REEP1; and GABRB3, optionally wherein said isolated population does not express EPCAM.

In some embodiments, said one or more markers are SUSD2; REEP1; and KCNB2, optionally wherein said isolated population does not express EPCAM.

In some embodiments, said one or more markers are SUSD2; REEP1; and GRM8, optionally wherein said isolated population does not express EPCAM.

In some embodiments, said one or more markers are SUSD2; REEP1; and DLL3, optionally wherein said isolated population does not express EPCAM.

In some embodiments, said one or more markers are SUSD2; REEP1; and KCNH8, optionally wherein said isolated population does not express EPCAM.

In some embodiments, said one or more markers are SUSD2; NPFFR2; and SORCS3, optionally wherein said isolated population does not express EPCAM.

In some embodiments, said one or more markers are SUSD2; NPFFR2; and NFASC, optionally wherein said isolated population does not express EPCAM.

In some embodiments, said one or more markers are SUSD2; NPFFR2; and GABRB3, optionally wherein said isolated population does not express EPCAM.

In some embodiments, said one or more markers are SUSD2; NPFFR2; and KCNB2, optionally wherein said isolated population does not express EPCAM.

In some embodiments, said one or more markers are SUSD2; NPFFR2; and GRM8, optionally wherein said isolated population does not express EPCAM.

In some embodiments, said one or more markers are SUSD2; NPFFR2; and DLL3, optionally wherein said isolated population does not express EPCAM.

In some embodiments, said one or more markers are SUSD2; NPFFR2; and KCNH8, optionally wherein said isolated population does not express EPCAM.

In some embodiments, said one or more markers are SUSD2; SORCS3; and NFASC, optionally wherein said isolated population does not express EPCAM.

In some embodiments, said one or more markers are SUSD2; SORCS3; and GABRB3, optionally wherein said isolated population does not express EPCAM.

In some embodiments, said one or more markers are SUSD2; SORCS3; and KCNB2, optionally wherein said isolated population does not express EPCAM.

In some embodiments, said one or more markers are SUSD2; SORCS3; and GRM8, optionally wherein said isolated population does not express EPCAM.

In some embodiments, said one or more markers are SUSD2; SORCS3; and DLL3, optionally wherein said isolated population does not express EPCAM.

In some embodiments, said one or more markers are SUSD2; SORCS3; and KCNH8, optionally wherein said isolated population does not express EPCAM.

In some embodiments, said one or more markers are SUSD2; NFASC; and GABRB3, optionally wherein said isolated population does not express EPCAM.

In some embodiments, said one or more markers are SUSD2; NFASC; and KCNB2, optionally wherein said isolated population does not express EPCAM.

In some embodiments, said one or more markers are SUSD2; NFASC; and GRM8, optionally wherein said isolated population does not express EPCAM.

In some embodiments, said one or more markers are SUSD2; NFASC; and DLL3, optionally wherein said isolated population does not express EPCAM.

In some embodiments, said one or more markers are SUSD2; NFASC; and KCNH8, optionally wherein said isolated population does not express EPCAM.

In some embodiments, said one or more markers are SUSD2; GABRB3; and KCNB2, optionally wherein said isolated population does not express EPCAM.

In some embodiments, said one or more markers are SUSD2; GABRB3; and GRM8, optionally wherein said isolated population does not express EPCAM.

In some embodiments, said one or more markers are SUSD2; GABRB3; and DLL3, optionally wherein said isolated population does not express EPCAM.

In some embodiments, said one or more markers are SUSD2; GABRB3; and KCNH8, optionally wherein said isolated population does not express EPCAM.

In some embodiments, said one or more markers are SUSD2; KCNB2; and GRM8, optionally wherein said isolated population does not express EPCAM.

In some embodiments, said one or more markers are SUSD2; KCNB2; and DLL3, optionally wherein said isolated population does not express EPCAM.

In some embodiments, said one or more markers are SUSD2; KCNB2; and KCNH8, optionally wherein said isolated population does not express EPCAM.

In some embodiments, said one or more markers are SUSD2; GRM8; and DLL3, optionally wherein said isolated population does not express EPCAM.

In some embodiments, said one or more markers are SUSD2; GRM8; and KCNH8, optionally wherein said isolated population does not express EPCAM.

In some embodiments, said one or more markers are SUSD2; DLL3; and KCNH8, optionally wherein said isolated population does not express EPCAM.

In some embodiments, said one or more markers are LPAR3; EPHA5; and CHRNA3, optionally wherein said isolated population does not express EPCAM.

In some embodiments, said one or more markers are LPAR3; EPHA5; and REEP1, optionally wherein said isolated population does not express EPCAM.

In some embodiments, said one or more markers are LPAR3; EPHA5; and NPFFR2, optionally wherein said isolated population does not express EPCAM.

In some embodiments, said one or more markers are LPAR3; EPHA5; and SORCS3, optionally wherein said isolated population does not express EPCAM.

In some embodiments, said one or more markers are LPAR3; EPHA5; and NFASC, optionally wherein said isolated population does not express EPCAM.

In some embodiments, said one or more markers are LPAR3; EPHA5; and GABRB3, optionally wherein said isolated population does not express EPCAM.

In some embodiments, said one or more markers are LPAR3; EPHA5; and KCNB2, optionally wherein said isolated population does not express EPCAM.

In some embodiments, said one or more markers are LPAR3; EPHA5; and GRM8, optionally wherein said isolated population does not express EPCAM.

In some embodiments, said one or more markers are LPAR3; EPHA5; and DLL3, optionally wherein said isolated population does not express EPCAM.

In some embodiments, said one or more markers are LPAR3; EPHA5; and KCNH8, optionally wherein said isolated population does not express EPCAM.

In some embodiments, said one or more markers are LPAR3; CHRNA3; and REEP1, optionally wherein said isolated population does not express EPCAM.

In some embodiments, said one or more markers are LPAR3; CHRNA3; and NPFFR2, optionally wherein said isolated population does not express EPCAM.

In some embodiments, said one or more markers are LPAR3; CHRNA3; and SORCS3, optionally wherein said isolated population does not express EPCAM.

In some embodiments, said one or more markers are LPAR3; CHRNA3; and NFASC, optionally wherein said isolated population does not express EPCAM.

In some embodiments, said one or more markers are LPAR3; CHRNA3; and GABRB3, optionally wherein said isolated population does not express EPCAM.

In some embodiments, said one or more markers are LPAR3; CHRNA3; and KCNB2, optionally wherein said isolated population does not express EPCAM.

In some embodiments, said one or more markers are LPAR3; CHRNA3; and GRM8, optionally wherein said isolated population does not express EPCAM.

In some embodiments, said one or more markers are LPAR3; CHRNA3; and DLL3, optionally wherein said isolated population does not express EPCAM.

In some embodiments, said one or more markers are LPAR3; CHRNA3; and KCNH8, optionally wherein said isolated population does not express EPCAM.

In some embodiments, said one or more markers are LPAR3; REEP1; and NPFFR2, optionally wherein said isolated population does not express EPCAM.

In some embodiments, said one or more markers are LPAR3; REEP1; and SORCS3, optionally wherein said isolated population does not express EPCAM.

In some embodiments, said one or more markers are LPAR3; REEP1; and NFASC, optionally wherein said isolated population does not express EPCAM.

In some embodiments, said one or more markers are LPAR3; REEP1; and GABRB3, optionally wherein said isolated population does not express EPCAM.

In some embodiments, said one or more markers are LPAR3; REEP1; and KCNB2, optionally wherein said isolated population does not express EPCAM.

In some embodiments, said one or more markers are LPAR3; REEP1; and GRM8, optionally wherein said isolated population does not express EPCAM.

In some embodiments, said one or more markers are LPAR3; REEP1; and DLL3, optionally wherein said isolated population does not express EPCAM.

In some embodiments, said one or more markers are LPAR3; REEP1; and KCNH8, optionally wherein said isolated population does not express EPCAM.

In some embodiments, said one or more markers are LPAR3; NPFFR2; and SORCS3, optionally wherein said isolated population does not express EPCAM.

In some embodiments, said one or more markers are LPAR3; NPFFR2; and NFASC, optionally wherein said isolated population does not express EPCAM.

In some embodiments, said one or more markers are LPAR3; NPFFR2; and GABRB3, optionally wherein said isolated population does not express EPCAM.

In some embodiments, said one or more markers are LPAR3; NPFFR2; and KCNB2, optionally wherein said isolated population does not express EPCAM.

In some embodiments, said one or more markers are LPAR3; NPFFR2; and GRM8, optionally wherein said isolated population does not express EPCAM.

In some embodiments, said one or more markers are LPAR3; NPFFR2; and DLL3, optionally wherein said isolated population does not express EPCAM.

In some embodiments, said one or more markers are LPAR3; NPFFR2; and KCNH8, optionally wherein said isolated population does not express EPCAM.

In some embodiments, said one or more markers are LPAR3; SORCS3; and NFASC, optionally wherein said isolated population does not express EPCAM.

In some embodiments, said one or more markers are LPAR3; SORCS3; and GABRB3, optionally wherein said isolated population does not express EPCAM.

In some embodiments, said one or more markers are LPAR3; SORCS3; and KCNB2, optionally wherein said isolated population does not express EPCAM.

In some embodiments, said one or more markers are LPAR3; SORCS3; and GRM8, optionally wherein said isolated population does not express EPCAM.

In some embodiments, said one or more markers are LPAR3; SORCS3; and DLL3, optionally wherein said isolated population does not express EPCAM.

In some embodiments, said one or more markers are LPAR3; SORCS3; and KCNH8, optionally wherein said isolated population does not express EPCAM.

In some embodiments, said one or more markers are LPAR3; NFASC; and GABRB3, optionally wherein said isolated population does not express EPCAM.

In some embodiments, said one or more markers are LPAR3; NFASC; and KCNB2, optionally wherein said isolated population does not express EPCAM.

In some embodiments, said one or more markers are LPAR3; NFASC; and GRM8, optionally wherein said isolated population does not express EPCAM.

In some embodiments, said one or more markers are LPAR3; NFASC; and DLL3, optionally wherein said isolated population does not express EPCAM.

In some embodiments, said one or more markers are LPAR3; NFASC; and KCNH8, optionally wherein said isolated population does not express EPCAM.

In some embodiments, said one or more markers are LPAR3; GABRB3; and KCNB2, optionally wherein said isolated population does not express EPCAM.

In some embodiments, said one or more markers are LPAR3; GABRB3; and GRM8, optionally wherein said isolated population does not express EPCAM.

In some embodiments, said one or more markers are LPAR3; GABRB3; and DLL3, optionally wherein said isolated population does not express EPCAM.

In some embodiments, said one or more markers are LPAR3; GABRB3; and KCNH8, optionally wherein said isolated population does not express EPCAM.

In some embodiments, said one or more markers are LPAR3; KCNB2; and GRM8, optionally wherein said isolated population does not express EPCAM.

In some embodiments, said one or more markers are LPAR3; KCNB2; and DLL3, optionally wherein said isolated population does not express EPCAM.

In some embodiments, said one or more markers are LPAR3; KCNB2; and KCNH8, optionally wherein said isolated population does not express EPCAM.

In some embodiments, said one or more markers are LPAR3; GRM8; and DLL3, optionally wherein said isolated population does not express EPCAM.

In some embodiments, said one or more markers are LPAR3; GRM8; and KCNH8, optionally wherein said isolated population does not express EPCAM.

In some embodiments, said one or more markers are LPAR3; DLL3; and KCNH8, optionally wherein said isolated population does not express EPCAM.

In some embodiments, said one or more markers are EPHA5; CHRNA3; and REEP1, optionally wherein said isolated population does not express EPCAM.

In some embodiments, said one or more markers are EPHA5; CHRNA3; and NPFFR2, optionally wherein said isolated population does not express EPCAM.

In some embodiments, said one or more markers are EPHA5; CHRNA3; and SORCS3, optionally wherein said isolated population does not express EPCAM.

In some embodiments, said one or more markers are EPHA5; CHRNA3; and NFASC, optionally wherein said isolated population does not express EPCAM.

In some embodiments, said one or more markers are EPHA5; CHRNA3; and GABRB3, optionally wherein said isolated population does not express EPCAM.

In some embodiments, said one or more markers are EPHA5; CHRNA3; and KCNB2, optionally wherein said isolated population does not express EPCAM.

In some embodiments, said one or more markers are EPHA5; CHRNA3; and GRM8, optionally wherein said isolated population does not express EPCAM.

In some embodiments, said one or more markers are EPHA5; CHRNA3; and DLL3, optionally wherein said isolated population does not express EPCAM.

In some embodiments, said one or more markers are EPHA5; CHRNA3; and KCNH8, optionally wherein said isolated population does not express EPCAM.

In some embodiments, said one or more markers are EPHA5; REEP1; and NPFFR2, optionally wherein said isolated population does not express EPCAM.

In some embodiments, said one or more markers are EPHA5; REEP1; and SORCS3, optionally wherein said isolated population does not express EPCAM.

In some embodiments, said one or more markers are EPHA5; REEP1; and NFASC, optionally wherein said isolated population does not express EPCAM.

In some embodiments, said one or more markers are EPHA5; REEP1; and GABRB3, optionally wherein said isolated population does not express EPCAM.

In some embodiments, said one or more markers are EPHA5; REEP1; and KCNB2, optionally wherein said isolated population does not express EPCAM.

In some embodiments, said one or more markers are EPHA5; REEP1; and GRM8, optionally wherein said isolated population does not express EPCAM.

In some embodiments, said one or more markers are EPHA5; REEP1; and DLL3, optionally wherein said isolated population does not express EPCAM.

In some embodiments, said one or more markers are EPHA5; REEP1; and KCNH8, optionally wherein said isolated population does not express EPCAM.

In some embodiments, said one or more markers are EPHA5; NPFFR2; and SORCS3, optionally wherein said isolated population does not express EPCAM.

In some embodiments, said one or more markers are EPHA5; NPFFR2; and NFASC, optionally wherein said isolated population does not express EPCAM.

In some embodiments, said one or more markers are EPHA5; NPFFR2; and GABRB3, optionally wherein said isolated population does not express EPCAM.

In some embodiments, said one or more markers are EPHA5; NPFFR2; and KCNB2, optionally wherein said isolated population does not express EPCAM.

In some embodiments, said one or more markers are EPHA5; NPFFR2; and GRM8, optionally wherein said isolated population does not express EPCAM.

In some embodiments, said one or more markers are EPHA5; NPFFR2; and DLL3, optionally wherein said isolated population does not express EPCAM.

In some embodiments, said one or more markers are EPHA5; NPFFR2; and KCNH8, optionally wherein said isolated population does not express EPCAM.

In some embodiments, said one or more markers are EPHA5; SORCS3; and NFASC, optionally wherein said isolated population does not express EPCAM.

In some embodiments, said one or more markers are EPHA5; SORCS3; and GABRB3, optionally wherein said isolated population does not express EPCAM.

In some embodiments, said one or more markers are EPHA5; SORCS3; and KCNB2, optionally wherein said isolated population does not express EPCAM.

In some embodiments, said one or more markers are EPHA5; SORCS3; and GRM8, optionally wherein said isolated population does not express EPCAM.

In some embodiments, said one or more markers are EPHA5; SORCS3; and DLL3, optionally wherein said isolated population does not express EPCAM.

In some embodiments, said one or more markers are EPHA5; SORCS3; and KCNH8, optionally wherein said isolated population does not express EPCAM.

In some embodiments, said one or more markers are EPHA5; NFASC; and GABRB3, optionally wherein said isolated population does not express EPCAM.

In some embodiments, said one or more markers are EPHA5; NFASC; and KCNB2, optionally wherein said isolated population does not express EPCAM.

In some embodiments, said one or more markers are EPHA5; NFASC; and GRM8, optionally wherein said isolated population does not express EPCAM.

In some embodiments, said one or more markers are EPHA5; NFASC; and DLL3, optionally wherein said isolated population does not express EPCAM.

In some embodiments, said one or more markers are EPHA5; NFASC; and KCNH8, optionally wherein said isolated population does not express EPCAM.

In some embodiments, said one or more markers are EPHA5; GABRB3; and KCNB2, optionally wherein said isolated population does not express EPCAM.

In some embodiments, said one or more markers are EPHA5; GABRB3; and GRM8, optionally wherein said isolated population does not express EPCAM.

In some embodiments, said one or more markers are EPHA5; GABRB3; and DLL3, optionally wherein said isolated population does not express EPCAM.

In some embodiments, said one or more markers are EPHA5; GABRB3; and KCNH8, optionally wherein said isolated population does not express EPCAM.

In some embodiments, said one or more markers are EPHA5; KCNB2; and GRM8, optionally wherein said isolated population does not express EPCAM.

In some embodiments, said one or more markers are EPHA5; KCNB2; and DLL3, optionally wherein said isolated population does not express EPCAM.

In some embodiments, said one or more markers are EPHA5; KCNB2; and KCNH8, optionally wherein said isolated population does not express EPCAM.

In some embodiments, said one or more markers are EPHA5; GRM8; and DLL3, optionally wherein said isolated population does not express EPCAM.

In some embodiments, said one or more markers are EPHA5; GRM8; and KCNH8, optionally wherein said isolated population does not express EPCAM.

In some embodiments, said one or more markers are EPHA5; DLL3; and KCNH8, optionally wherein said isolated population does not express EPCAM.

In some embodiments, said one or more markers are CHRNA3; REEP1; and NPFFR2, optionally wherein said isolated population does not express EPCAM.

In some embodiments, said one or more markers are CHRNA3; REEP1; and SORCS3, optionally wherein said isolated population does not express EPCAM.

In some embodiments, said one or more markers are CHRNA3; REEP1; and NFASC, optionally wherein said isolated population does not express EPCAM.

In some embodiments, said one or more markers are CHRNA3; REEP1; and GABRB3, optionally wherein said isolated population does not express EPCAM.

In some embodiments, said one or more markers are CHRNA3; REEP1; and KCNB2, optionally wherein said isolated population does not express EPCAM.

In some embodiments, said one or more markers are CHRNA3; REEP1; and GRM8, optionally wherein said isolated population does not express EPCAM.

In some embodiments, said one or more markers are CHRNA3; REEP1; and DLL3, optionally wherein said isolated population does not express EPCAM.

In some embodiments, said one or more markers are CHRNA3; REEP1; and KCNH8, optionally wherein said isolated population does not express EPCAM.

In some embodiments, said one or more markers are CHRNA3; NPFFR2; and SORCS3, optionally wherein said isolated population does not express EPCAM.

In some embodiments, said one or more markers are CHRNA3; NPFFR2; and NFASC, optionally wherein said isolated population does not express EPCAM.

In some embodiments, said one or more markers are CHRNA3; NPFFR2; and GABRB3, optionally wherein said isolated population does not express EPCAM.

In some embodiments, said one or more markers are CHRNA3; NPFFR2; and KCNB2, optionally wherein said isolated population does not express EPCAM.

In some embodiments, said one or more markers are CHRNA3; NPFFR2; and GRM8, optionally wherein said isolated population does not express EPCAM.

In some embodiments, said one or more markers are CHRNA3; NPFFR2; and DLL3, optionally wherein said isolated population does not express EPCAM.

In some embodiments, said one or more markers are CHRNA3; NPFFR2; and KCNH8, optionally wherein said isolated population does not express EPCAM.

In some embodiments, said one or more markers are CHRNA3; SORCS3; and NFASC, optionally wherein said isolated population does not express EPCAM.

In some embodiments, said one or more markers are CHRNA3; SORCS3; and GABRB3, optionally wherein said isolated population does not express EPCAM.

In some embodiments, said one or more markers are CHRNA3; SORCS3; and KCNB2, optionally wherein said isolated population does not express EPCAM.

In some embodiments, said one or more markers are CHRNA3; SORCS3; and GRM8, optionally wherein said isolated population does not express EPCAM.

In some embodiments, said one or more markers are CHRNA3; SORCS3; and DLL3, optionally wherein said isolated population does not express EPCAM.

In some embodiments, said one or more markers are CHRNA3; SORCS3; and KCNH8, optionally wherein said isolated population does not express EPCAM.

In some embodiments, said one or more markers are CHRNA3; NFASC; and GABRB3, optionally wherein said isolated population does not express EPCAM.

In some embodiments, said one or more markers are CHRNA3; NFASC; and KCNB2, optionally wherein said isolated population does not express EPCAM.

In some embodiments, said one or more markers are CHRNA3; NFASC; and GRM8, optionally wherein said isolated population does not express EPCAM.

In some embodiments, said one or more markers are CHRNA3; NFASC; and DLL3, optionally wherein said isolated population does not express EPCAM.

In some embodiments, said one or more markers are CHRNA3; NFASC; and KCNH8, optionally wherein said isolated population does not express EPCAM.

In some embodiments, said one or more markers are CHRNA3; GABRB3; and KCNB2, optionally wherein said isolated population does not express EPCAM.

In some embodiments, said one or more markers are CHRNA3; GABRB3; and GRM8, optionally wherein said isolated population does not express EPCAM.

In some embodiments, said one or more markers are CHRNA3; GABRB3; and DLL3, optionally wherein said isolated population does not express EPCAM.

In some embodiments, said one or more markers are CHRNA3; GABRB3; and KCNH8, optionally wherein said isolated population does not express EPCAM.

In some embodiments, said one or more markers are CHRNA3; KCNB2; and GRM8, optionally wherein said isolated population does not express EPCAM.

In some embodiments, said one or more markers are CHRNA3; KCNB2; and DLL3, optionally wherein said isolated population does not express EPCAM.

In some embodiments, said one or more markers are CHRNA3; KCNB2; and KCNH8, optionally wherein said isolated population does not express EPCAM.

In some embodiments, said one or more markers are CHRNA3; GRM8; and DLL3, optionally wherein said isolated population does not express EPCAM.

In some embodiments, said one or more markers are CHRNA3; GRM8; and KCNH8, optionally wherein said isolated population does not express EPCAM.

In some embodiments, said one or more markers are CHRNA3; DLL3; and KCNH8, optionally wherein said isolated population does not express EPCAM.

In some embodiments, said one or more markers are REEP1; NPFFR2; and SORCS3, optionally wherein said isolated population does not express EPCAM.

In some embodiments, said one or more markers are REEP1; NPFFR2; and NFASC, optionally wherein said isolated population does not express EPCAM.

In some embodiments, said one or more markers are REEP1; NPFFR2; and GABRB3, optionally wherein said isolated population does not express EPCAM.

In some embodiments, said one or more markers are REEP1; NPFFR2; and KCNB2, optionally wherein said isolated population does not express EPCAM.

In some embodiments, said one or more markers are REEP1; NPFFR2; and GRM8, optionally wherein said isolated population does not express EPCAM.

In some embodiments, said one or more markers are REEP1; NPFFR2; and DLL3, optionally wherein said isolated population does not express EPCAM.

In some embodiments, said one or more markers are REEP1; NPFFR2; and KCNH8, optionally wherein said isolated population does not express EPCAM.

In some embodiments, said one or more markers are REEP1; SORCS3; and NFASC, optionally wherein said isolated population does not express EPCAM.

In some embodiments, said one or more markers are REEP1; SORCS3; and GABRB3, optionally wherein said isolated population does not express EPCAM.

In some embodiments, said one or more markers are REEP1; SORCS3; and KCNB2, optionally wherein said isolated population does not express EPCAM.

In some embodiments, said one or more markers are REEP1; SORCS3; and GRM8, optionally wherein said isolated population does not express EPCAM.

In some embodiments, said one or more markers are REEP1; SORCS3; and DLL3, optionally wherein said isolated population does not express EPCAM.

In some embodiments, said one or more markers are REEP1; SORCS3; and KCNH8, optionally wherein said isolated population does not express EPCAM.

In some embodiments, said one or more markers are REEP1; NFASC; and GABRB3, optionally wherein said isolated population does not express EPCAM.

In some embodiments, said one or more markers are REEP1; NFASC; and KCNB2, optionally wherein said isolated population does not express EPCAM.

In some embodiments, said one or more markers are REEP1; NFASC; and GRM8, optionally wherein said isolated population does not express EPCAM.

In some embodiments, said one or more markers are REEP1; NFASC; and DLL3, optionally wherein said isolated population does not express EPCAM.

In some embodiments, said one or more markers are REEP1; NFASC; and KCNH8, optionally wherein said isolated population does not express EPCAM.

In some embodiments, said one or more markers are REEP1; GABRB3; and KCNB2, optionally wherein said isolated population does not express EPCAM.

In some embodiments, said one or more markers are REEP1; GABRB3; and GRM8, optionally wherein said isolated population does not express EPCAM.

In some embodiments, said one or more markers are REEP1; GABRB3; and DLL3, optionally wherein said isolated population does not express EPCAM.

In some embodiments, said one or more markers are REEP1; GABRB3; and KCNH8, optionally wherein said isolated population does not express EPCAM.

In some embodiments, said one or more markers are REEP1; KCNB2; and GRM8, optionally wherein said isolated population does not express EPCAM.

In some embodiments, said one or more markers are REEP1; KCNB2; and DLL3, optionally wherein said isolated population does not express EPCAM.

In some embodiments, said one or more markers are REEP1; KCNB2; and KCNH8, optionally wherein said isolated population does not express EPCAM.

In some embodiments, said one or more markers are REEP1; GRM8; and DLL3, optionally wherein said isolated population does not express EPCAM.

In some embodiments, said one or more markers are REEP1; GRM8; and KCNH8, optionally wherein said isolated population does not express EPCAM.

In some embodiments, said one or more markers are REEP1; DLL3; and KCNH8, optionally wherein said isolated population does not express EPCAM.

In some embodiments, said one or more markers are NPFFR2; SORCS3; and NFASC, optionally wherein said isolated population does not express EPCAM.

In some embodiments, said one or more markers are NPFFR2; SORCS3; and GABRB3, optionally wherein said isolated population does not express EPCAM.

In some embodiments, said one or more markers are NPFFR2; SORCS3; and KCNB2, optionally wherein said isolated population does not express EPCAM.

In some embodiments, said one or more markers are NPFFR2; SORCS3; and GRM8, optionally wherein said isolated population does not express EPCAM.

In some embodiments, said one or more markers are NPFFR2; SORCS3; and DLL3, optionally wherein said isolated population does not express EPCAM.

In some embodiments, said one or more markers are NPFFR2; SORCS3; and KCNH8, optionally wherein said isolated population does not express EPCAM.

In some embodiments, said one or more markers are NPFFR2; NFASC; and GABRB3, optionally wherein said isolated population does not express EPCAM.

In some embodiments, said one or more markers are NPFFR2; NFASC; and KCNB2, optionally wherein said isolated population does not express EPCAM.

In some embodiments, said one or more markers are NPFFR2; NFASC; and GRM8, optionally wherein said isolated population does not express EPCAM.

In some embodiments, said one or more markers are NPFFR2; NFASC; and DLL3, optionally wherein said isolated population does not express EPCAM.

In some embodiments, said one or more markers are NPFFR2; NFASC; and KCNH8, optionally wherein said isolated population does not express EPCAM.

In some embodiments, said one or more markers are NPFFR2; GABRB3; and KCNB2, optionally wherein said isolated population does not express EPCAM.

In some embodiments, said one or more markers are NPFFR2; GABRB3; and GRM8, optionally wherein said isolated population does not express EPCAM.

In some embodiments, said one or more markers are NPFFR2; GABRB3; and DLL3, optionally wherein said isolated population does not express EPCAM.

In some embodiments, said one or more markers are NPFFR2; GABRB3; and KCNH8, optionally wherein said isolated population does not express EPCAM.

In some embodiments, said one or more markers are NPFFR2; KCNB2; and GRM8, optionally wherein said isolated population does not express EPCAM.

In some embodiments, said one or more markers are NPFFR2; KCNB2; and DLL3, optionally wherein said isolated population does not express EPCAM.

In some embodiments, said one or more markers are NPFFR2; KCNB2; and KCNH8, optionally wherein said isolated population does not express EPCAM.

In some embodiments, said one or more markers are NPFFR2; GRM8; and DLL3, optionally wherein said isolated population does not express EPCAM.

In some embodiments, said one or more markers are NPFFR2; GRM8; and KCNH8, optionally wherein said isolated population does not express EPCAM.

In some embodiments, said one or more markers are NPFFR2; DLL3; and KCNH8, optionally wherein said isolated population does not express EPCAM.

In some embodiments, said one or more markers are SORCS3; NFASC; and GABRB3, optionally wherein said isolated population does not express EPCAM.

In some embodiments, said one or more markers are SORCS3; NFASC; and KCNB2, optionally wherein said isolated population does not express EPCAM.

In some embodiments, said one or more markers are SORCS3; NFASC; and GRM8, optionally wherein said isolated population does not express EPCAM.

In some embodiments, said one or more markers are SORCS3; NFASC; and DLL3, optionally wherein said isolated population does not express EPCAM.

In some embodiments, said one or more markers are SORCS3; NFASC; and KCNH8, optionally wherein said isolated population does not express EPCAM.

In some embodiments, said one or more markers are SORCS3; GABRB3; and KCNB2, optionally wherein said isolated population does not express EPCAM.

In some embodiments, said one or more markers are SORCS3; GABRB3; and GRM8, optionally wherein said isolated population does not express EPCAM.

In some embodiments, said one or more markers are SORCS3; GABRB3; and DLL3, optionally wherein said isolated population does not express EPCAM.

In some embodiments, said one or more markers are SORCS3; GABRB3; and KCNH8, optionally wherein said isolated population does not express EPCAM.

In some embodiments, said one or more markers are SORCS3; KCNB2; and GRM8, optionally wherein said isolated population does not express EPCAM.

In some embodiments, said one or more markers are SORCS3; KCNB2; and DLL3, optionally wherein said isolated population does not express EPCAM.

In some embodiments, said one or more markers are SORCS3; KCNB2; and KCNH8, optionally wherein said isolated population does not express EPCAM.

In some embodiments, said one or more markers are SORCS3; GRM8; and DLL3, optionally wherein said isolated population does not express EPCAM.

In some embodiments, said one or more markers are SORCS3; GRM8; and KCNH8, optionally wherein said isolated population does not express EPCAM.

In some embodiments, said one or more markers are SORCS3; DLL3; and KCNH8, optionally wherein said isolated population does not express EPCAM.

In some embodiments, said one or more markers are NFASC; GABRB3; and KCNB2, optionally wherein said isolated population does not express EPCAM.

In some embodiments, said one or more markers are NFASC; GABRB3; and GRM8, optionally wherein said isolated population does not express EPCAM.

In some embodiments, said one or more markers are NFASC; GABRB3; and DLL3, optionally wherein said isolated population does not express EPCAM.

In some embodiments, said one or more markers are NFASC; GABRB3; and KCNH8, optionally wherein said isolated population does not express EPCAM.

In some embodiments, said one or more markers are NFASC; KCNB2; and GRM8, optionally wherein said isolated population does not express EPCAM.

In some embodiments, said one or more markers are NFASC; KCNB2; and DLL3, optionally wherein said isolated population does not express EPCAM.

In some embodiments, said one or more markers are NFASC; KCNB2; and KCNH8, optionally wherein said isolated population does not express EPCAM.

In some embodiments, said one or more markers are NFASC; GRM8; and DLL3, optionally wherein said isolated population does not express EPCAM.

In some embodiments, said one or more markers are NFASC; GRM8; and KCNH8, optionally wherein said isolated population does not express EPCAM.

In some embodiments, said one or more markers are NFASC; DLL3; and KCNH8, optionally wherein said isolated population does not express EPCAM.

In some embodiments, said one or more markers are GABRB3; KCNB2; and GRM8, optionally wherein said isolated population does not express EPCAM.

In some embodiments, said one or more markers are GABRB3; KCNB2; and DLL3, optionally wherein said isolated population does not express EPCAM.

In some embodiments, said one or more markers are GABRB3; KCNB2; and KCNH8, optionally wherein said isolated population does not express EPCAM.

In some embodiments, said one or more markers are GABRB3; GRM8; and DLL3, optionally wherein said isolated population does not express EPCAM.

In some embodiments, said one or more markers are GABRB3; GRM8; and KCNH8, optionally wherein said isolated population does not express EPCAM.

In some embodiments, said one or more markers are GABRB3; DLL3; and KCNH8, optionally wherein said isolated population does not express EPCAM.

In some embodiments, said one or more markers are KCNB2; GRM8; and DLL3, optionally wherein said isolated population does not express EPCAM.

In some embodiments, said one or more markers are KCNB2; GRM8; and KCNH8, optionally wherein said isolated population does not express EPCAM.

In some embodiments, said one or more markers are KCNB2; DLL3; and KCNH8, optionally wherein said isolated population does not express EPCAM.

In some embodiments, said one or more markers are GRM8; DLL3; and KCNH8, optionally wherein said isolated population does not express EPCAM.

### In-vitro cell population

As described herein, the present invention provides an in-vitro cell population comprising spiral ganglion neural progenitors (SGNPs) that can be utilized in both medical research and therapeutic applications. This SGNP cell population, which may be provided in a vial, represents a valuable resource for regenerative medicine and auditory research, as these progenitor cells have the capacity to differentiate into spiral ganglion neurons, the essential cells responsible for transmitting auditory signals to the brain.

In some embodiments, the in-vitro cell population is derived from pluripotent stem cells (hPSC). In some embodiments, the in-vitro cell population is derived from human induced pluripotent stem cells (hiPSCs).

In some embodiments, the SGNPs are mammalian SGNPs, such as murine, canine, feline, lagomorph, primate, preferably human SGNPs.

In some embodiments, the in-vitro cell population is for use in medicine.

In some embodiments, the in-vitro cell population is for use in drug screening.

As used herein, the term "drug screening" may refer to a process by which compounds are tested to assess their biological activity, efficacy, or toxicity in a systematic manner. Drug screening may involve evaluating large libraries of chemical compounds to identify those that have a desired therapeutic effect or biological activity against a particular target, such as a protein, cell type, or pathogen. This process can be conducted through in vitro assays, in vivo models, or high-throughput automated systems that allow for the rapid evaluation of numerous compounds.

In some embodiments, the in-vitro cell population is for use in cell therapy. As used herein, the term "cell therapy" may refer to a therapeutic approach involving the administration of living cells to restore, replace, or enhance the function of damaged or diseased tissues. This may encompass the use of autologous cells (derived from the patient), allogeneic cells (derived from a donor), or other cell sources, including stem cells, immune cells, or genetically modified cells. The cells may be processed, expanded, or activated ex vivo prior to administration. Cell therapy may be applied in the treatment of various conditions, including but not limited to hearing loss and associated disorders, cancer, autoimmune diseases, degenerative disorders, and injuries.

In some embodiments, said population comprises at least 50% SGNPs, such as at least 60% SGNPs, such as at least 75% SGNPs, such as at least 80% SGNPs, such as at least 90% SGNPs, such as at least 95% SGNPs, such as at least 99% SGNPs, such as at least 99,5% SGNPs.

In some embodiments, said population comprises at least 1 million cells, such as at at least 5 million cells, such as at least 10 million cells, such as at least 50 million cells, such as at least 100 million cells, such as at least 1 billion cells.

The present invention relates further to a vial comprising cell culture medium and an in-vitro cell population as described herein. The terms "Medium", "Cell culture medium", "Culture medium" as used herein refer to a solution containing nutrients that nourish growing cells. In certain embodiments, the culture medium is useful for growing mammalian cells. Typically, a culture medium provides essential and non-essential amino acids, vitamins, energy sources, lipids, and trace elements required by the cell for minimal growth and/or survival. A culture medium may also contain supplementary components that enhance growth and/or survival above the minimal rate, including, but not limited to, hormones and/or other growth factors, particular ions (such as sodium, chloride, calcium, magnesium, and phosphate), buffers, vitamins, nucleosides or nucleotides, trace elements (inorganic compounds usually present at very low final concentrations), amino acids, lipids, and/or glucose or other energy source. In some embodiments, the culture medium contains differentiation factors.

In some embodiments, the vial comprising cell culture medium and an in-vitro cell population comprises at least 1 million cells, such as at least 5 million cells, such as at least 10 million cells, such as at least 50 million cells, such as at least 100 million cells, such as at least 1 billion cells. In other embodiments, the in-vitro cell population comprising spiral ganglion neural progenitors (SGNPs) is at a concentration of at least 1 million cells, such as at least 5 million cells, such as at least 10 million cells, such as at least 50 million cells, such as at least 100 million cells, such as at least 1 billion cells per mL

### Isolated cell population

As described herein, the present invention provides an isolated cell population comprising spiral ganglion neural progenitors (SGNPs) that can be utilized in both medical research and therapeutic applications. This cell population, which may be provided in a vial, represents a valuable resource for regenerative medicine and auditory research, as these progenitor cells have the capacity to differentiate into spiral ganglion neurons, the essential cells responsible for transmitting auditory signals to the brain.

In some embodiments, the isolated cell population is derived from pluripotent stem cells (hPSC). In some embodiments, the isolated cell population is derived from human induced pluripotent stem cells (hiPSCs).

In some embodiments, the SGNPs are mammalian SGNPs, such as murine, canine, feline, lagomorph, primate, preferably human SGNPs.

In some embodiments, the isolated cell population is for use in medicine.

In some embodiments, the isolated cell population is for use in drug screening.

As used herein, the term "drug screening" may refer to a process by which compounds are tested to assess their biological activity, efficacy, or toxicity in a systematic manner. Drug screening may involve evaluating large libraries of chemical compounds to identify those that have a desired therapeutic effect or biological activity against a particular target, such as a protein, cell type, or pathogen. This process can be conducted through in vitro assays, in vivo models, or high-throughput automated systems that allow for the rapid evaluation of numerous compounds.

In some embodiments, the isolated cell population is for use in cell therapy. As used herein, the term "cell therapy" may refer to a therapeutic approach involving the administration of living cells to restore, replace, or enhance the function of damaged or diseased tissues. This may encompass the use of autologous cells (derived from the patient), allogeneic cells (derived from a donor), or other cell sources, including stem cells, immune cells, or genetically modified cells. The cells may be processed, expanded, or activated ex vivo prior to administration. Cell therapy may be applied in the treatment of various conditions, including but not limited to hearing loss and associated disorders, cancer, autoimmune diseases, degenerative disorders, and injuries.

In some embodiments, said population comprises at least 50% SGNPs, such as at least 60% SGNPs, such as at least 75% SGNPs, such as at least 80% SGNPs, such as at least 90% SGNPs, such as at least 95% SGNPs, such as at least 99% SGNPs, such as at least 99,5% SGNPs.

In some embodiments, said population comprises at least 1 million cells, such as at at least 5 million cells, such as at least 10 million cells, such as at least 50 million cells, such as at least 100 million cells, such as at least 1 billion cells.

The present invention relates further to a vial comprising cell culture medium and isolated cell population as described herein. The terms "Medium", "Cell culture medium", "Culture medium" as used herein refer to a solution containing nutrients that nourish growing cells. In certain embodiments, the culture medium is useful for growing mammalian cells. Typically, a culture medium provides essential and non-essential amino acids, vitamins, energy sources, lipids, and trace elements required by the cell for minimal growth and/or survival. A culture medium may also contain supplementary components that enhance growth and/or survival above the minimal rate, including, but not limited to, hormones and/or other growth factors, particular ions (such as sodium, chloride, calcium, magnesium, and phosphate), buffers, vitamins, nucleosides or nucleotides, trace elements (inorganic compounds usually present at very low final concentrations), amino acids, lipids, and/or glucose or other energy source. In some embodiments, the culture medium contains differentiation factors.

In some embodiments, the vial comprising cell culture medium and an isolated cell population comprises at least 1 million cells, such as at least 5 million cells, such as at least 10 million cells, such as at least 50 million cells, such as at least 100 million cells, such as at least 1 billion cells. In other embodiments, the isolated cell population comprising spiral ganglion neural progenitors (SGNPs) is at a concentration of at least 1 million cells, such as at least 5 million cells, such as at least 10 million cells, such as at least 50 million cells, such as at least 100 million cells, such as at least 1 billion cells per mL

### Differentiation of SGNPs

As described herein, the invention relates to a cell population comprising spiral ganglion neural progenitors (SGNPs) that can be efficiently differentiated into auditory neurons, such as Type I and Type II spiral ganglion neurons. These neurons can play a critical role in the auditory pathway, with Type I neurons transmitting the majority of auditory signals from inner hair cells to the brain, while Type II neurons are involved in conveying input from outer hair cells, contributing to sound modulation and sensitivity. An improved differentiation efficiency can make the cell population particularly valuable for auditory research, drug screening, and the development of regenerative therapies aimed at restoring or improving hearing function.

In some embodiments, the SGNPs have the ability to efficiently differentiate to spiral ganglion neurons.

As used herein, the term "sensory cells" may refer to fully differentiated cells that are specialized in detecting and transmitting sensory information, such as sound, light, touch, or chemical signals, to the nervous system. These cells include, but are not limited to, auditory hair cells in the inner ear, photoreceptor cells in the retina, olfactory receptor cells in the nose, and mechanoreceptors in the skin. Mature sensory cells are responsible for converting external stimuli into electrical signals, which are then relayed to the brain for processing and interpretation. Their proper function is critical for sensory perception, and damage to these cells can lead to sensory impairments such as hearing loss, blindness, or loss of tactile sensation. Sensory cells, such as auditory hair cells in the inner ear, can be identified by the expression of markers that are specific to their function in detecting stimuli. The person skilled in the art will appreciate that markers for auditory hair cells are known and include among others Myosin VIIa,

Prestin and Atoh1 (Math1). In some embodiments, the sensory cells are auditory hair cells, such as inner hair cells and/or outer hair cells, or vestibular hair cells.

As used herein, the term "supporting cells" in the ear may refer to non-sensory cells located within the cochlea and other parts of the inner ear that play crucial roles in maintaining the function and integrity of auditory hair cells. These cells can provide structural support and help to maintain the ion balance within the cochlear environment. Supporting cells in the ear may also serve as a barrier, protecting sensory hair cells from mechanical and chemical damage. Supporting cells in the cochlea or other sensory organs may be distinguished by markers that reflect their non-sensory, structural, and protective roles. The person skilled in the art will appreciate that markers for supporting cells are known and include among others Sox2, P27kip1 and Glial fibrillary acidic protein (GFAP). In some embodiments, the supporting cells are pillar cells, Deiters' cells, Hensens's cells, Claudius cells and/or phalangeal cells.

As used herein, the term "neurons" may refer to specialized cells within the auditory system that are responsible for transmitting sound information from the ear to the brain. In the ear, these neurons, specifically the spiral ganglion neurons (SGNs), can be located in the cochlea and can play a crucial role in hearing. They may consist of three main parts: the cell body (soma), which contains the nucleus and genetic material; dendrites, which receive auditory signals from the sensory hair cells in the cochlea; and an axon, which transmits these signals to the auditory nerve, ultimately relaying the information to the brain for processing. The person skilled in the art will appreciate that markers for neurons, such as spiral ganglion neurons are known and include among others NeuN (Neuronal Nuclei), Tuj1 (Beta III Tubulin), Neurofilament and Brn3a (POU4F1). In some embodiments, the neurons are auditory neurons, such as Type I spiral ganglion neurons and/or Type II spiral ganglion neurons, vestibular neurons and/ or efferent neurons, such as medial olivocochlear neurons and/or lateral olivocochlear neurons.

During differentiation, a cell undergoes changes in gene expression, protein synthesis, and morphology, allowing it to perform specific roles within an organism. The person skilled in the art will appreciate, that differentiation can be determined by analysing specific markers, such as the expression of lineage-specific genes and proteins, changes in cell morphology, or the presence of distinct functional traits associated with a particular cell type. The process is regulated by genetic signals and environmental factors, and once differentiation occurs, cells typically retain their specialized state to contribute to the overall function of tissues and organs. For instance, differentiation of cells to neurons can be determined by analysing markers specific to neurons, while differentiation of cells to sensory cells can be determined by analysing markers specific to sensory cells.

In some embodiments of the present invention, the undifferentiated cells are partially-differentiated cells. In some embodiments, the undifferentiated cells are not cancer cells. In some embodiments, the undifferentiated cells are cells that grow, and divide controlled by the normal regulatory mechanisms that govern cell division, growth, and death. In some embodiments of the present invention, the differentiated cells are partially-differentiated cells. In some embodiments of the present invention, the differentiated cells are terminally-differentiated cells.

Partially-differentiated or lineage-committed cells can be called progenitor cells or precursor cells. Progenitor cells can be cells that have begun the process of differentiation, meaning they have started to acquire specific characteristics and functions of a particular cell type but have not yet fully completed this process. These cells may exist in a transitional state between multipotent stem cells and fully differentiated cells, retaining some potential to differentiate further. For example, they may express certain markers or perform some functions of their target cell type but are not yet fully specialized. Unipotent cells or monopotent cells can form a single differentiated cell lineage. Undifferentiated cells are cells, as defined herein, are all cells, which are not terminally differentiated. Terminally differentiated cells, as defined herein, are cells that lost their ability to proliferate permanently, although they continue to perform their specialized functions for a long time.

In some embodiments, at least 5%, such as at least 10%, such as at least 15%, such as at least 25%, such as at least 40%, such as at least 50%, such as at least 75%, such as at least 85%, such as at least 90%, such as at least 95% of the SGNPs differentiate to spiral ganglion neurons.

### Characteristics of the cell population comprising SGNPs

As described herein, the cell population comprising spiral ganglion neural progenitor (SGNP) possesses several distinct characteristics that can make it highly suitable for therapeutic applications. One feature of this population comprising SGNP can be its high reproducibility during differentiation, consistently yielding well-defined populations of auditory neurons across experiments. This reproducibility can be quantitatively assessed through metrics such as cell yield and qualitative markers like morphological characteristics. For example, the nucleus-to-cytoplasm ratio, a key indicator of cellular maturation, can be measured using microscopy-based techniques or flow cytometry, providing a reliable measure of differentiation progress. The consistency in achieving uniform and reproducible cell populations, along with their clear morphological features, underscores the potential of this SGNP cell population for use in drug screening, regenerative therapies, and auditory neuron research.

In some embodiments, when the cells are differentiated to spiral ganglion neurons, the method of differentiation achieves reproducibility, such as consistent results across multiple differentiation attempts as measured by at least one parameter of cell yield, marker expression levels, and/or morphological characteristics.

In some embodiments, the cell yield is at least 1 million viable SGNPs, such as at least 5 million viable SGNPs, such as at least 10 million viable SGNPs, such as at least 50 million viable SGNPs, such as at least 100 million viable SGNPs, such as at least 1 billion viable SGNPs per cm², wherein the viable cells express one or more markers of CXCR4, SUSD2, LPAR3, EPHA5, CHRNA3, REEP1, NPFFR2, SORCS3, NFASC, GABRB3, KCNB2, GRM8, DLL3, and/or KCNH8.

In some embodiments, the marker expression levels are measured by one or more of Flow cytometry, immunocytochemistry, immunofluorescence, western blotting, quantitative Real-time PCR, ELISA, Mass spectroscopy, and RNA sequencing. Preferably the marker expression is measured using a non-destructive technique, such as Flow cytometry or other types of cytometry. As used herein, the term "reproducibility" may refer to the ability to achieve consistent and identical results across multiple independent repetitions of an experimental procedure, using the same methodology, data sets, and conditions. Reproducibility is a critical metric for evaluating the reliability of experimental findings, ensuring that observed outcomes are not artifacts of variability or bias in the experimental setup. In technical terms, reproducibility can be assessed by statistical analysis of the variance between trials.

As used herein, the term "cell yield" may refer to the total number of viable cells obtained from a biological sample or culture following a specific isolation, extraction, or expansion procedure. Cell yield is an important parameter in both experimental and therapeutic contexts, indicating the efficiency of a given protocol in harvesting or producing cells. It can be quantified as the number of cells per unit volume and may be influenced by factors such as cell type, growth conditions, and the efficiency of the isolation technique.

In some embodiments, the morphological characteristics are one or more of shape, Nucleus-to-Cytoplasm ratio and cell surface. As used herein, the term "morphological characteristics" may refer to the observable structural and physical features of cells. These characteristics may include size, shape, organization, and specific structural components such as the presence of certain organelles or surface features. In a cellular context, morphological characteristics can help distinguish between different cell types, states, or stages of differentiation. These traits can be assessed through various techniques, such as microscopy (light, electron, or fluorescence), FACS or sequencing.

In some embodiments, the marker expression level of one or more of Myo7a, Espin, Atoh1, Sox2, Jagged1, P27kip1, SOX2, TUJ1, Brn3a and Neurofilament is measured.

### Methods to identify, isolate and/or enrich SGNPs

As described herein, the invention provides a method for identifying spiral ganglion neural progenitors (SGNPs) from an in-vitro mixed cell population, such as an otic progenitor cells population. Further, as described herein, the invention provides a method for identifying spiral ganglion neural progenitors (SGNPs) from an isolated mixed cell population, such as an otic progenitor cells population. This method leverages the expression of specific molecular markers, which can be detected using techniques like immunostaining, flow cytometry, or quantitative PCR. By using these markers, this method allows for the reliable identification and isolation of SGNPs from heterogeneous cell populations, ensuring a highly enriched source of progenitor cells for downstream applications such as differentiation studies, drug screening, and regenerative medicine efforts targeting auditory neuron regeneration.

In some embodiments, the in-vitro cell population is derived from pluripotent stem cells (hPSC). In some embodiments, the in-vitro cell population is derived from human induced pluripotent stem cells (hiPSCs).

In some embodiments, the isolated cell population is derived from pluripotent stem cells (hPSC). In some embodiments, the in-vitro cell population is derived from human induced pluripotent stem cells (hiPSCs).

Thus, the present invention relates further to a method to identify spiral ganglion neural progenitors (SGNPs) from an in-vitro mixed cell population, the method comprising the step of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population;
thereby identifying a population comprising SGNPs from the in-vitro mixed population.

Thus, the present invention relates further to a method to identify spiral ganglion neural progenitors (SGNPs) from an isolated mixed cell population, the method comprising the step of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population;
thereby identifying a population comprising SGNPs from the isolated mixed population.

The present invention relates further to a method to isolate spiral ganglion neural progenitors (SGNPs) from an in-vitro mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
b. Isolating the cells expressing at least one of the markers;
thereby isolating a population comprising SGNPs from the in-vitro mixed population.

The present invention relates further to a method to isolate spiral ganglion neural progenitors (SGNPs) from an isolated mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population; and
b. Isolating the cells expressing at least one of the markers;
thereby isolating a population comprising SGNPs from the in-vitro mixed population.

The present invention relates further to a method to enrich spiral ganglion neural progenitors (SGNPs) from an in-vitro mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
b. Isolating the cells expressing at least one of the markers;
thereby enriching a population comprising SGNPs from the in-vitro mixed population.

The present invention relates further to a method to enrich spiral ganglion neural progenitors (SGNPs) from an isolated mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population; and
b. Isolating the cells expressing at least one of the markers;
thereby enriching a population of SGNPs from the isolated mixed population.

The present invention relates further to a method to obtain a population of cells enriched with spiral ganglion neural progenitors (SGNPs) from an in-vitro mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
b. Isolating the cells expressing at least one of the markers;
thereby enriching a population of SGNPs from the in-vitro mixed population.

The present invention relates further to a method to obtain a population of cells enriched with spiral ganglion neural progenitors (SGNPs) from an isolated mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population; and
b. Isolating the cells expressing at least one of the markers;
thereby enriching a population of SGNPs from the isolated mixed population.

In some embodiments, said at least one marker are SUSD2, LPAR3, EPHA5, CHRNA3, REEP1, and/or NPFFR2.

In some embodiments, said at least one marker are SUSD2, LPAR3, CHRNA3, REEP1, and/or NPFFR2.

In some embodiments, said at least one marker are CXCR4,SORCS3, NFASC, GABRB3, KCNB2, and/or GRM8.

In some embodiments, said at least one marker are DLL3 and/or KCNH8.

In some embodiments, said at least one marker is CXCR4.

In some embodiments, said at least one marker are cell-surface markers.

In some embodiments, the method comprises further a step of detecting the absence of expression of a cell-surface marker, preferably EPCAM, on single cells of the in-vitro mixed cell population.

In some embodiments, the step of detecting the absence of expression of the cell-surface marker is performed before, simultaneously, and/or after, the step of detecting the expression of the at least one marker.

In some embodiments, the method comprises further a step of labelling cells expressing the at least one marker.

In some embodiments, the labelling is with one or more labelled binding members arranged to bind the at least one marker, preferably wherein the labelled binding members are fluorescently labelled antibody(ies), nanobodies or microbodies specific for the one or more markers, or magnetic beads conjugated to antibody(ies), nanobodies or microbodies specific for the at least one marker. As used herein, the term "labelled binding members" may refer to molecules or agents that are modified with a detectable label and are capable of binding specifically to target molecules, such as proteins, nucleic acids, or other biomolecules. These binding members may include antibodies, ligands, aptamers, or peptides that are designed to recognize and attach to a specific target with high affinity. The label can be a fluorescent dye, radioactive isotope, enzyme, or other detectable marker that allows the binding event to be monitored or quantified. Labelled binding members are commonly used in techniques such as immunoassays, flow cytometry, Western blotting, and imaging, where they facilitate the detection, localization, or quantification of specific molecules within a biological sample.

The person skilled in the art will appreciate that different plating protocols can be used dependent on the cell type and cell lineage. Furthermore, different cell culture plates can be used, such as multi-well plates preferably containing glass coverslips.

The person skilled in the art will also appreciate, that the cells can be seeded at different densities depending on the cell type.

In some embodiments, the method comprises further a step of sorting from said in-vitro mixed cell population the cells in which said expression of marker(s) is detected.

In some embodiments, the method comprises further a step of sorting from said isolated mixed cell population the cells in which said expression of marker(s) is detected.

In some embodiments, the method comprises further a step of sorting from said in-vitro mixed cell population the cells in which said absence of cell-surface marker expression, such as EPCAM expression, is detected.

In some embodiments, the method comprises further a step of sorting from said isolated mixed cell population the cells in which said absence of cell-surface marker expression, such as EPCAM expression, is detected.

In some embodiments, the step of sorting from said in-vitro mixed cell population the cells in which said expression of markers is detected is performed before, during, and/or after, the step of sorting from said in-vitro mixed cell population the cells in which said absence of cell-surface marker expression, such as EPCAM expression is detected.

In some embodiments, the step of sorting from said isolated mixed cell population the cells in which said expression of markers is detected is performed before, during, and/or after, the step of sorting from said isolated mixed cell population the cells in which said absence of cell-surface marker expression, such as EPCAM expression is detected.

In some embodiments, the step of detection of the expression of the at least one marker is performed by immunocytochemistry or flow cytometry.

In some embodiments, the step of detection of the expression and/or the step of sorting the cells is performed by flow cytometry, such as fluorescence activated cell sorting (FACS), Magnetic Activated Cell Sorting (MACS), or microfluidic cell sorting.

In some embodiments,
a. the marker CXCR4 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
b. the marker SUSD2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
c. the marker LPAR3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
d. the marker EPHA5 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
e. the marker CHRNA3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
f. the marker REEP1 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
g. the marker NPFFR2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
h. the marker SORCS3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
i. the marker NFASC is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
j. the marker GABRB3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
k. the marker KCNB2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
l. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
m. the marker KCNH8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population; and/or
n. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the in-vitro mixed population;
after isolating the cells expressing the at least one marker.

In some embodiments, the marker EPCAM is expressed in at most 99%, such as at most 90% such as at most 75%, such as at most 50%, such as at most 25%, such as at most 20%, such as at most 15%, such as at most 10%, such as at most 7.5%, such as at most 5%, such as at most 4%, such as at most 3%, such as at most 2%, such as at most 1% of the cells after isolating the cells expressing of the at least one marker.

In some embodiments, the cell population comprising spiral ganglion neural progenitors (SGNPs identified from a cell population by the method as described herein is as described in section "SGNP marker".

### Methods to obtain SGNPs

As described herein, the invention provides a method to obtain spiral ganglion neural progenitors (SGNPs) by differentiating human induced pluripotent stem cells (iPSCs) into SGNPs and sorting them based on specific cell markers. This method involves a multi-step cell culture process where iPSCs are progressively exposed to different culture media and growth factors to promote neural lineage commitment and auditory progenitor specification. Key signalling pathways are modulated during each stage of differentiation, guiding the iPSCs towards an SGNP fate. In some embodiments, SGNPs are identified and isolated using fluorescence-activated cell sorting (FACS) or magnetic-activated cell sorting (MACS) based on the expression markers as described herein. The present invention relates further an in-vitro cell population comprising spiral ganglion neural progenitors (SGNPs) obtained by the method comprising following steps:
a. Plating human pluripotent stem cells;
b. Culturing the pluripotent stem cells in medium;
c. Differentiating of the pluripotent stem cells in medium promoting the differentiation to otic progenitor cells;
d. Differentiating the cells of step c) in medium activating BMP signalling;
e. Culturing the cells of step d) in medium comprising a BMP signal inhibitor and bFGF; and
f. Proliferating the cells of step e) to obtain SGNPs;
g. isolating cells from the population of step f) that exhibit one or more marker is CXCR4, SUSD2, LPAR3, EPHA5, CHRNA3, REEP1, NPFFR2, SORCS3, CXCR4, NFASC, GABRB3, KCNB2, GRM8, DLL3, and/or KCNH8.

In some embodiments, the pluripotent stem cells are cultured before plating the cells in step a). In the context of the present disclosure, the terms "cultivation" of cells and "cell culture" are to be interpreted broadly, such that they encompass for example conditions in which cells divide, differentiate and/or proliferate, situations in which cells are maintained in a differentiated state with retention of at least one functional characteristic exhibited by the cell type when present in its natural environment, and situations in which stem cells are maintained in an undifferentiated state. As used herein, the term "human pluripotent stem cells" (hPSCs) may refer to stem cells derived from human tissues that have the capacity to differentiate into nearly all cell types of the body. These cells are characterized by their ability to self-renew indefinitely and their pluripotency, which means they can give rise to cells from the three primary germ layers: ectoderm, mesoderm, and endoderm. Human pluripotent stem cells include embryonic stem cells (ESCs), derived from the inner cell mass of the blastocyst, and induced pluripotent stem cells (iPSCs), which are reprogrammed from adult somatic cells to a pluripotent state. In all embodiments, the stem cell is not obtained by methods that involve the use of human embryos for commercial or industrial purposes. In all embodiments, the stem cell is not obtained by methods that necessarily involve the destruction of a human embryo.

In some embodiments, the cultured pluripotent stem cells are dissociated upon reaching 90% confluency, such as dissociated using TrypLE. By "confluence", in the present invention, it is meant that the growth surface of the culture is being uniformly covered by a single layer of cells.

In some embodiments, the pluripotent stem cells in step a) are plated at a cell density of from 10 000 cells/cm² to 30 000 cells/cm², such as from 12 500 cells/cm² to 25 000 cells/cm², such as from 15 000 cells/cm² to 20 000 cells/cm², such as 18.000 cells/m².

In some embodiments, the pluripotent stem cells in step b) are cultured in stem cell medium.

In some embodiments, the pluripotent stem cells in step b) are cultured for at least 1 day, such as 2 days.

In some embodiments, the pluripotent stem cells in step c) are cultured in medium comprising DMEM/F12, B27, N2, NEAA, BME, Penicillin-Streptomycin and a TGF-β inhibitor. As used herein, the term "TGF-β inhibitor" may refer to a molecule or compound that blocks or reduces the activity of the Transforming Growth Factor Beta (TGF-β) signalling pathway. TGF-β inhibitors may function by targeting different components of the pathway, such as the TGF-β ligands themselves, their receptors (TGF-βRI or TGF-βRII), or downstream signalling molecules like SMAD proteins.

In some embodiments, the pluripotent stem cells in step c) are cultured for at least 1 days, such as 2 days.

In some embodiments, the pluripotent stem cells in step d) are cultured in medium comprising DMEM/F12, B27, N2, NEAA, BME, Penicillin-Streptomycin and BMP4.

In some embodiments, the pluripotent stem cells in step d) are cultured for at least 1 day, such as at least 2 days, such as 3 days.

In some embodiments, the pluripotent stem cells in step e) are cultured in medium comprising DMEM/F12, B27, N2, NEAA, BME, Penicillin-Streptomycin, bFGF and a BMP inhibitor.

In some embodiments, the pluripotent stem cells in step e) are cultured for at least 1 day, such as at least 2 days, such as 3 days, such as at least 5 days, such as at least 10 days, such as at least 13 days.

In some embodiments, the pluripotent stem cells in step f) are cultured in medium comprising DMEM/F12, B27, N2, NEAA, BME, Penicillin-Streptomycin, bFGF, IGF-1 and dbcAMP.

In some embodiments of the methods of the present invention, the in-vitro mixed cell population is an otic progenitor cells (OPCs) population.

### Kit

The present invention relates further to a kit for isolating the in-vitro population comprising spiral ganglion neural progenitors (SGNPs) as described herein, said kit comprising:
a. labelled binding members arranged to bind one or more markers from CXCR4, SUSD2, LPAR3, EPHA5, CHRNA3, REEP1, NPFFR2, SORCS3, NFASC, GABRB3, KCNB2, GRM8, DLL3 and/or KCNH8, preferably wherein said binding members are antibody(ies), nanobodies or microbodies specific for said markers, or magnetic beads conjugated to antibody(ies), nanobodies or microbodies specific for said markers;
b. Instructions performing the method as described herein.

The present invention relates further to a kit for isolating the isolated population comprising spiral ganglion neural progenitors (SGNPs) as described herein, said kit comprising:
a. labelled binding members arranged to bind one or more markers from CXCR4, SUSD2, LPAR3, EPHA5, CHRNA3, REEP1, NPFFR2, SORCS3, NFASC, GABRB3, KCNB2, GRM8, DLL3 and/or KCNH8, preferably wherein said binding members are antibody(ies), nanobodies or microbodies specific for said markers, or magnetic beads conjugated to antibody(ies), nanobodies or microbodies specific for said markers;
b. Instructions performing the method as described herein.

### Items

1. An in-vitro cell population comprising spiral ganglion neural progenitors (SGNPs), wherein said population expresses one or more markers, wherein the one or more marker is CXCR4, SUSD2, LPAR3, EPHA5, CHRNA3, REEP1, NPFFR2, SORCS3, NFASC, GABRB3, KCNB2, GRM8, DLL3, and/or KCNH8.
2. The in-vitro cell population according to item 1, wherein said population expresses one or more markers, wherein the one or more marker is CXCR4, SUSD2, LPAR3, CHRNA3, REEP1, NPFFR2, SORCS3, NFASC, GABRB3, KCNB2, GRM8, DLL3, and/or KCNH8.
3. The in-vitro cell population according to any one of the preceding items, wherein said one or more markers are SUSD2, LPAR3, EPHA5, CHRNA3, REEP1, and/or NPFFR2.
4. The in-vitro cell population according to any one of the preceding items, wherein said one or more markers are SUSD2, LPAR3, CHRNA3, REEP1, and/or NPFFR2.
5. The in-vitro cell population according to any one of the preceding items, wherein said one or more markers are CXCR4, SORCS3, NFASC, GABRB3, KCNB2, and/or GRM8.
6. The in-vitro cell population according to any one of the preceding items, wherein said one or more markers are DLL3 and/or KCNH8.
7. The in-vitro cell population according to any one of the preceding items, wherein said one or more markers is CXCR4.
8. The in-vitro cell population according to any one of the preceding items, wherein said population does not express EPCAM.
9. The in-vitro cell population according to any one of the preceding items, wherein:
   a. the marker CXCR4 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   b. the marker SUSD2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   c. the marker LPAR3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   d. the marker EPHA5 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   e. the marker CHRNA3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   f. the marker REEP1 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   g. the marker NPFFR2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   h. the marker SORCS3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   i. the marker NFASC is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   j. the marker GABRB3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   k. the marker KCNB2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   l. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   m. the marker KCNH8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population; and/or
   n. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population.
10. The in-vitro cell population according to any one of the preceding items, wherein the marker EPCAM is expressed in at most 99%, such as at most 90% such as at most 75%, such as at most 50%, such as at most 25%, such as at most 20%, such as at most 15%, such as at most 10%, such as at most 7.5%, such as at most 5%, such as at most 4%, such as at most 3%, such as at most 2%, such as at most 1%, such as 0% of the cells of the population.
11. The in-vitro cell population according to any one of the preceding items, wherein said population further expresses ISL1, POU4F1, and/or NEUROD1.
12. The in-vitro cell population according to any one of the preceding items, wherein the SGNPs are mammalian SGNPs, such as murine, canine, feline, lagomorph, primate, preferably human SGNPs.
13. The in-vitro cell population according to any one of the preceding items, for use in medicine.
14. The in-vitro cell population according to any one of the preceding items for use in drug screening.
15. The in-vitro cell population according to any one of items 1 to 13 for use in cell therapy.
16. The in-vitro cell population according to any one of the preceding items, wherein said population comprises at least 50% SGNPs, such as at least 60% SGNPs, such as at least 75% SGNPs, such as at least 80% SGNPs, such as at least 90% SGNPs, such as at least 95% SGNPs, such as at least 99% SGNPs, such as at least 99,5% SGNPs.
17. The in-vitro cell population according to any one of the preceding items, wherein the SGNPs have the ability to efficiently differentiate to spiral ganglion neurons (SGNs).
18. The in-vitro cell population according to item 17, wherein the spiral ganglion neurons are Type I spiral ganglion neurons and/or Type II spiral ganglion neurons.
19. The in-vitro cell population according to any one of items 17 to 18, wherein at least 5%, such as at least 10%, such as at least 15%, such as at least 25%, such as at least 40%, such as at least 50%, such as at least 75%, such as at least 85%, such as at least 90%, such as at least 95% of the SGNPs differentiate to spiral ganglion neurons.
20. The in-vitro cell population comprising SGNPs according to any one of items 17 to 19, wherein when the cells are differentiated to spiral ganglion neurons, the method of differentiation achieves reproducibility, such as consistent results across multiple differentiation attempts as measured by at least one parameter of cell yield, marker expression levels, and/or morphological characteristics.
21. The in-vitro cell population comprising SGNPs according to item 20, wherein the marker expression levels are measured by one or more of Flow cytometry, immunocytochemistry, immunofluorescence, western blotting, quantitative Real-time PCR, ELISA, Mass spectroscopy, and RNA sequencing.
22. The in-vitro cell population comprising SGNPs according to item 20, wherein the morphological characteristics are one or more of shape, Nucleus-to-Cytoplasm ratio and cell surface.
23. The in-vitro cell population comprising SGNPs according to any one of items 20 to 22, wherein the marker expression level of one or more of Myo7a, Espin, Atoh1, Sox2, Jagged1, P27kip1, SOX2, TUJ1, Brn3a and Neurofilament is measured.
24. The in-vitro cell population comprising SGNPs according to any one of the preceding items, wherein the population is derived from hPSCs, such as hiPSCs.
25. A method to identify spiral ganglion neural progenitors (SGNPs) from an in-vitro mixed cell population, the method comprising the step of:
   a. Detecting expression of at least one marker of , CXCR4,SUSD2, LPAR3, EPHA5, CHRNA3, REEP1, NPFFR2, SORCS3 NFASC, GABRB3, KCNB2, GRM8, DLL3 and KCNH8 on single cells of the in-vitro mixed cell population;
   thereby identifying a population comprising SGNPs from the in-vitro mixed population.
26. A method to isolate spiral ganglion neural progenitors (SGNPs) from an in-vitro mixed cell population, the method comprising the steps of:
   a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
   b. Isolating the cells expressing at least one of the markers;
   thereby isolating a population comprising SGNPs from the in-vitro mixed population.
27. A method to enrich spiral ganglion neural progenitors (SGNPs) from an in-vitro mixed cell population, the method comprising the steps of:
   a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
   b. Isolating the cells expressing at least one of the markers;
   thereby enriching a population of SGNPs from the in-vitro mixed population.
28. The method according to any one of items 25 to 27, wherein said at least one marker are SUSD2, LPAR3, EPHA5, CHRNA3, REEP1, and/or NPFFR2.
29. The method according to any one of items 25 to 27, wherein said at least one marker are SUSD2, LPAR3, CHRNA3, REEP1, and/or NPFFR2.
30. The method according to any one of items 25 to 29, wherein said at least one marker are CXCR4, SORCS3, NFASC, GABRB3, KCNB2, and/or GRM8.
31. The method according to any one of items 25 to 30, wherein said at least one marker are DLL3 and/or KCNH8.
32. The method according to any one of items 25 to 31, wherein said at least one marker is CXCR4.
33. The method according to any one of items 25 to 32, wherein said at least one marker are cell-surface markers.
34. The method according to any one of items 25 to 33, further comprising a step of detecting the absence of expression of a cell-surface marker, preferably EPCAM, on single cells of the in-vitro mixed cell population.
35. The method according to item 34, wherein the step of detecting the absence of expression of the cell-surface marker is performed before, simultaneously, and/or after, the step of detecting the expression of the at least one marker.
36. The method according to any one of items 25 to 35, further comprising a step of labelling cells expressing the at least one marker.
37. The method according to item 36, wherein the labelling is with one or more labelled binding members arranged to bind the at least one marker, preferably wherein the labelled binding members are fluorescently labelled antibody(ies), nanobodies or microbodies specific for the one or more markers, or magnetic beads conjugated to antibody(ies), nanobodies or microbodies specific for the at least one marker.
38. The method according to any one of items 25 and 28 to 37, further comprising a step of sorting from said in-vitro mixed cell population the cells in which said expression of marker(s) is detected.
39. The method according to any one of items 33 to 38, further comprising a step of sorting from said in-vitro mixed cell population the cells in which said absence of cell-surface marker expression, such as EPCAM expression, is detected.
40. The method according to any one of items 38 to 39, wherein the step of sorting from said in-vitro mixed cell population the cells in which said expression of markers is detected is performed before, during, and/or after, the step of sorting from said in-vitro mixed cell population the cells in which said absence of cell-surface marker expression, such as EPCAM expression is detected.
41. The method according to any one of items 25 to 40, wherein the step of detection of the expression of the at least one marker is performed by immunocytochemistry or flow cytometry.
42. The method according to any one of items 25 to 41, wherein the step of detection of the expression and/or the step of sorting the cells is performed by flow cytometry, such as fluorescence activated cell sorting (FACS), Magnetic Activated Cell Sorting (MACS), or microfluidic cell sorting.
43. The method according to any one of items 26 to 42, wherein:
   a. the marker CXCR4 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   b. the marker SUSD2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   c. the marker LPAR3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   d. the marker EPHA5 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   e. the marker CHRNA3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   f. the marker REEP1 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   g. the marker NPFFR2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   h. the marker SORCS3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   i. the marker NFASC is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   j. the marker GABRB3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   k. the marker KCNB2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   l. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   m. the marker KCNH8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population; and/or
   n. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the in-vitro mixed cell population;
   after isolating the cells expressing the at least one marker.
44. The method according to any one of items 26 to 43, wherein the marker EPCAM is expressed in at most 99%, such as at most 90% such as at most 75%, such as at most 50%, such as at most 25%, such as at most 20%, such as at most 15%, such as at most 10%, such as at most 7.5%, such as at most 5%, such as at most 4%, such as at most 3%, such as at most 2%, such as at most 1% of the cells after isolating the cells expressing of the at least one marker.
45. The method according to any one of items 25 to 44, wherein spiral ganglion neural progenitors (SGNPs) are as described in any one of items 1 to 23.
46. An in-vitro cell population comprising spiral ganglion neural progenitors (SGNPs) obtained by the method comprising following steps:
   a. Plating human pluripotent stem cells;
   b. Culturing the pluripotent stem cells in medium;
   c. Differentiating of the pluripotent stem cells in medium promoting the differentiation to otic progenitor cells;
   d. Differentiating the cells of step c) in medium activating BMP signaling;
   e. Culturing the cells of step d) in medium comprising a BMP signal inhibitor and bFGF; and
   f. Proliferating the cells of step e) to obtain SGNPs;
   g. isolating cells from the population of step f) that exhibit one or more marker is CXCR4, SUSD2, LPAR3, EPHA5, CHRNA3, REEP1, NPFFR2, SORCS3, CXCR4, NFASC, GABRB3, KCNB2, GRM8, DLL3, and/or KCNH8.
47. The in-vitro cell population according to item 46, wherein the pluripotent stem cells are cultured before plating the cells in step a).
48. The in-vitro cell population according to item 46, wherein the cultured pluripotent stem cells are dissociated upon reaching 90% confluency, such as dissociated using TrypLE.
49. The in-vitro cell population according to any one of items 46 to 48, wherein the pluripotent stem cells in step a) are plated at a cell density of from 10 000 cells/cm² to 30 000 cells/cm², such as from 12 500 cells/cm² to 25 000 cells/cm², such as from 15 000 cells/cm² to 20 000 cells/cm², such as 18.000 cells/m².
50. The in-vitro cell population according to any one of items 46 to 49, wherein the pluripotent stem cells in step b) are cultured in medium supporting the culture of stem cells supplemented with ROCK inhibitor, such as with 10 µM ROCK inhibitor.
51. The in-vitro cell population according to any one of items 46 to 50, wherein the pluripotent stem cells in step b) are cultured for at least 1 day, such as 2 days.
52. The in-vitro cell population according to any one of items 46 to 51, wherein the pluripotent stem cells in step c) are cultured in medium comprising DMEM/F12, B27, N2, NEAA, BME, Penicillin-Streptomycin and a TGF-β inhibitor, such as SB431542.
53. The in-vitro cell population according to any one of items 46 to 52, wherein the pluripotent stem cells in step c) are cultured for at least 1 days, such as 2 days.
54. The in-vitro cell population according to any one of items 46 to 53, wherein the pluripotent stem cells in step d) are cultured in medium comprising DMEM/F12, B27, N2, NEAA, BME, Penicillin-Streptomycin and BMP4.
55. The in-vitro cell population according to any one of items 46 to 54, wherein the pluripotent stem cells in step d) are cultured for at least 1 day, such as at least 2 days, such as 3 days.
56. The in-vitro cell population according to any one of items 46 to 55, wherein the pluripotent stem cells in step e) are cultured in medium comprising DMEM/F12, B27, N2, NEAA, BME, Penicillin-Streptomycin, bFGF and a BMP inhibitor.
57. The in-vitro cell population according to any one of items 46 to 56, wherein the pluripotent stem cells in step e) are cultured for at least 1 day, such as at least 2 days, such as 3 days, such as at least 5 days, such as at least 10 days, such as at least 13 days.
58. The in-vitro cell population according to any one of items 46 to 57, wherein the pluripotent stem cells in step f) are cultured in medium comprising DMEM/F12, B27, N2, NEAA, BME, Penicillin-Streptomycin, bFGF, IGF-1 and dbcAMP.
59. The in-vitro cell population according to any one of items 1 to 23, or the method according to any one of items 25 to 45, wherein the in-vitro cell population or the in-vitro mixed cell population is the in-vitro cell population of any one of items 49 to 61.
60. The method according to any one of items 25 to 59, wherein the in-vitro mixed cell population is an otic progenitors cells (OPCs) population.
61. A kit for isolating the in-vitro population comprising spiral ganglion neural progenitors (SGNPs) according to any one of items 1 to 23, said kit comprising :
   a. labelled binding members arranged to bind one or more markers from CXCR4, SUSD2, LPAR3, EPHA5, CHRNA3, REEP1, NPFFR2, SORCS3, NFASC, GABRB3, KCNB2, GRM8, DLL3 and/or KCNH8, preferably wherein said binding members are antibody(ies), nanobodies or microbodies specific for said markers, or magnetic beads conjugated to antibody(ies), nanobodies or microbodies specific for said markers;
   b. Instructions performing the method according to any one of items 25 to 60.
62. A vial comprising cell culture medium and an in-vitro cell population according to any one of items 1 to 23.
63. The in-vitro cell population, the method, or the vial according to any one of the preceding items, wherein the in vitro-cell population comprising spiral ganglion neural progenitors (SGNPs) comprises at least 1 million cells, such as at least 5 million cells, such as at least 10 million cells, such as at least 50 million cells, such as at least 100 million cells, such as at least 1 billion cells, for example at a concentration of at least 1 million cells, such as at least 5 million cells, such as at least 10 million cells, such as at least 50 million cells, such as at least 100 million cells, such as at least 1 billion cells per mL.
64. The in-vitro cell population, the method, or the vial according to any one of the preceding items, wherein the in vitro-cell population comprising spiral ganglion neural progenitors (SGNPs) comprises at least 1 million SGNPs, such as at least 5 million SGNPs, such as at least 10 million SGNPs, such as at least 50 million SGNPs, such as at least 100 million SGNPs, such as at least 1 billion SGNPs, for example at a concentration of at least 1 million SGNPs, such as at least 5 million SGNPs, such as at least 10 million SGNPs, such as at least 50 million SGNPs, such as at least 100 million SGNPs, such as at least 1 billion SGNPs per mL.

### Items 2

1. An isolated cell population comprising spiral ganglion neural progenitors (SGNPs), wherein said population expresses one or more markers, wherein the one or more marker is CXCR4, SUSD2, LPAR3, EPHA5, CHRNA3, REEP1, NPFFR2, SORCS3, NFASC, GABRB3, KCNB2, GRM8, DLL3, and/or KCNH8.
2. The isolated cell population according to item 1, wherein said population expresses one or more markers, wherein the one or more marker is CXCR4, SUSD2, LPAR3, CHRNA3, REEP1, NPFFR2, SORCS3, NFASC, GABRB3, KCNB2, GRM8, DLL3, and/or KCNH8.
3. The isolated cell population according to any one of the preceding items, wherein said one or more markers are SUSD2, LPAR3, EPHA5, CHRNA3, REEP1, and/or NPFFR2.
4. The isolated cell population according to any one of the preceding items, wherein said one or more markers are SUSD2, LPAR3, CHRNA3, REEP1, and/or NPFFR2.
5. The isolated cell population according to any one of the preceding items, wherein said one or more markers are CXCR4, SORCS3, NFASC, GABRB3, KCNB2, and/or GRM8.
6. The isolated cell population according to any one of the preceding items, wherein said one or more markers are DLL3 and/or KCNH8.
7. The isolated cell population according to any one of the preceding items, wherein said one or more markers is CXCR4.
8. The isolated cell population according to any one of the preceding items, wherein said population does not express EPCAM.
9. The isolated cell population according to any one of the preceding items, wherein:
   a. the marker CXCR4 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   b. the marker SUSD2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   c. the marker LPAR3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   d. the marker EPHA5 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   e. the marker CHRNA3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   f. the marker REEP1 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   g. the marker NPFFR2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   h. the marker SORCS3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   i. the marker NFASC is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   j. the marker GABRB3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   k. the marker KCNB2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   l. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   m. the marker KCNH8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population; and/or
   n. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population.
10. The isolated cell population according to any one of the preceding items, wherein the marker EPCAM is expressed in at most 99%, such as at most 90% such as at most 75%, such as at most 50%, such as at most 25%, such as at most 20%, such as at most 15%, such as at most 10%, such as at most 7.5%, such as at most 5%, such as at most 4%, such as at most 3%, such as at most 2%, such as at most 1%, such as 0% of the cells of the population.
11. The isolated cell population according to any one of the preceding items, wherein said population further expresses ISL1, POU4F1, and/or NEUROD1.
12. The isolated cell population according to any one of the preceding items, wherein the SGNPs are mammalian SGNPs, such as murine, canine, feline, lagomorph, primate, preferably human SGNPs.
13. The isolated cell population according to any one of the preceding items, for use in medicine.
14. The isolated cell population according to any one of the preceding items for use in drug screening.
15. The isolated cell population according to any one of items 1 to 13 for use in cell therapy.
16. The isolated cell population according to any one of the preceding items, wherein said population comprises at least 50% SGNPs, such as at least 60% SGNPs, such as at least 75% SGNPs, such as at least 80% SGNPs, such as at least 90% SGNPs, such as at least 95% SGNPs, such as at least 99% SGNPs, such as at least 99,5% SGNPs.
17. The isolated cell population according to any one of the preceding items, wherein the SGNPs have the ability to efficiently differentiate to spiral ganglion neurons (SGNs).
18. The isolated cell population according to item 17, wherein the spiral ganglion neurons are Type I spiral ganglion neurons and/or Type II spiral ganglion neurons.
19. The isolated cell population according to any one of items 17 to 18, wherein at least 5%, such as at least 10%, such as at least 15%, such as at least 25%, such as at least 40%, such as at least 50%, such as at least 75%, such as at least 85%, such as at least 90%, such as at least 95% of the SGNPs differentiate to spiral ganglion neurons.
20. The isolated cell population comprising SGNPs according to any one of items 17 to 19, wherein when the cells are differentiated to spiral ganglion neurons, the method of differentiation achieves reproducibility, such as consistent results across multiple differentiation attempts as measured by at least one parameter of cell yield, marker expression levels, and/or morphological characteristics.
21. The isolated cell population SGNPs according to item 20, wherein the marker expression levels are measured by one or more of Flow cytometry, immunocytochemistry, immunofluorescence, western blotting, quantitative Real-time PCR, ELISA, Mass spectroscopy, and RNA sequencing.
22. The isolated cell population comprising SGNPs according to item 20, wherein the morphological characteristics are one or more of shape, Nucleus-to-Cytoplasm ratio and cell surface.
23. The isolated cell population comprising SGNPs according to any one of items 20 to 22, wherein the marker expression level of one or more of Myo7a, Espin, Atoh1, Sox2, Jagged1, P27kip1, SOX2, TUJ1, Brn3a and Neurofilament is measured.
24. The in-vitro cell population comprising SGNPs according to any one of the preceding items, wherein the population is derived from hPSCs, such as hiPSCs.
25. A method to identify spiral ganglion neural progenitors (SGNPs) from an isolated mixed cell population, the method comprising the step of:
   a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population;
   thereby identifying a population comprising SGNPs from the isolated mixed population.
26. A method to isolate spiral ganglion neural progenitors (SGNPs) from an isolated mixed cell population, the method comprising the steps of:
   a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population; and
   b. Isolating the cells expressing at least one of the markers;
   thereby isolating a population comprising SGNPs from the isolated mixed population.
27. A method to enrich spiral ganglion neural progenitors (SGNPs) from an isolated mixed cell population, the method comprising the steps of:
   a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the isolated mixed cell population; and
   b. Isolating the cells expressing at least one of the markers;
   thereby enriching a population of SGNPs from the isolated mixed population.
28. The method according to any one of items 25 to 27, wherein said at least one marker are SUSD2, LPAR3, EPHA5, CHRNA3, REEP1, and/or NPFFR2.
29. The method according to any one of items 25 to 27, wherein said at least one marker are SUSD2, LPAR3, CHRNA3, REEP1, and/or NPFFR2.
30. The method according to any one of items 25 to 29, wherein said at least one marker are CXCR4, SORCS3, NFASC, GABRB3, KCNB2, and/or GRM8.
31. The method according to any one of items 25 to 30, wherein said at least one marker are DLL3 and/or KCNH8.
32. The method according to any one of items 25 to 31, wherein said at least one marker is CXCR4.
33. The method according to any one of items 25 to 32, wherein said at least one marker are cell-surface markers.
34. The method according to any one of items 25 to 33, further comprising a step of detecting the absence of expression of a cell-surface marker, preferably EPCAM, on single cells of the in-vitro mixed cell population.
35. The method according to item 34, wherein the step of detecting the absence of expression of the cell-surface marker is performed before, simultaneously, and/or after, the step of detecting the expression of the at least one marker.
36. The method according to any one of items 25 to 35, further comprising a step of labelling cells expressing the at least one marker.
37. The method according to item 36, wherein the labelling is with one or more labelled binding members arranged to bind the at least one marker, preferably wherein the labelled binding members are fluorescently labelled antibody(ies), nanobodies or microbodies specific for the one or more markers, or magnetic beads conjugated to antibody(ies), nanobodies or microbodies specific for the at least one marker.
38. The method according to any one of items 25 and 28 to 37, further comprising a step of sorting from said isolated mixed cell population the cells in which said expression of marker(s) is detected.
39. The method according to any one of items 33 to 38, further comprising a step of sorting from said isolated mixed cell population the cells in which said absence of cell-surface marker expression, such as EPCAM expression, is detected.
40. The method according to any one of items 38 to 39, wherein the step of sorting from said isolated mixed cell population the cells in which said expression of markers is detected is performed before, during, and/or after, the step of sorting from said isolated mixed cell population the cells in which said absence of cell-surface marker expression, such as EPCAM expression is detected.
41. The method according to any one of items 25 to 40, wherein the step of detection of the expression of the at least one marker is performed by immunocytochemistry or flow cytometry.
42. The method according to any one of items 25 to 41, wherein the step of detection of the expression and/or the step of sorting the cells is performed by flow cytometry, such as fluorescence activated cell sorting (FACS), Magnetic Activated Cell Sorting (MACS), or microfluidic cell sorting.
43. The method according to any one of items 26 to 42, wherein:
   a. the marker CXCR4 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   b. the marker SUSD2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   c. the marker LPAR3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   d. the marker EPHA5 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   e. the marker CHRNA3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   f. the marker REEP1 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   g. the marker NPFFR2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   h. the marker SORCS3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   i. the marker NFASC is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   j. the marker GABRB3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   k. the marker KCNB2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   l. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
   m. the marker KCNH8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population; and/or
   n. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the in-vitro mixed cell population;
   after isolating the cells expressing the at least one marker.
44. The method according to any one of items 26 to 43, wherein the marker EPCAM is expressed in at most 99%, such as at most 90% such as at most 75%, such as at most 50%, such as at most 25%, such as at most 20%, such as at most 15%, such as at most 10%, such as at most 7.5%, such as at most 5%, such as at most 4%, such as at most 3%, such as at most 2%, such as at most 1% of the cells after isolating the cells expressing of the at least one marker.
45. The method according to any one of items 25 to 44, wherein spiral ganglion neural progenitors (SGNPs) are as described in any one of items 1 to 23.
46. An isolated cell population comprising spiral ganglion neural progenitors (SGNPs) obtained by the method comprising following steps:
   a. Plating human pluripotent stem cells;
   b. Culturing the pluripotent stem cells in medium;
   c. Differentiating of the pluripotent stem cells in medium promoting the differentiation to otic progenitor cells;
   d. Differentiating the cells of step c) in medium activating BMP signaling;
   e. Culturing the cells of step d) in medium comprising a BMP signal inhibitor and bFGF; and
   f. Proliferating the cells of step e) to obtain SGNPs;
   g. isolating cells from the population of step f) that exhibit one or more marker is CXCR4, SUSD2, LPAR3, EPHA5, CHRNA3, REEP1, NPFFR2, SORCS3, CXCR4, NFASC, GABRB3, KCNB2, GRM8, DLL3, and/or KCNH8.
47. The isolated cell population according to item 46, wherein the pluripotent stem cells are cultured before plating the cells in step a).
48. The isolated cell population according to item 46, wherein the cultured pluripotent stem cells are dissociated upon reaching 90% confluency, such as dissociated using TrypLE.
49. The isolated cell population according to any one of items 46 to 48, wherein the pluripotent stem cells in step a) are plated at a cell density of from 10 000 cells/cm² to 30 000 cells/cm², such as from 12 500 cells/cm² to 25 000 cells/cm², such as from 15 000 cells/cm² to 20 000 cells/cm², such as 18.000 cells/m².
50. The isolated cell population according to any one of items 46 to 49, wherein the pluripotent stem cells in step b) are cultured in medium supporting the culture of stem cells supplemented with ROCK inhibitor, such as with 10 µM ROCK inhibitor.
51. The isolated cell population according to any one of items 46 to 50, wherein the pluripotent stem cells in step b) are cultured for at least 1 day, such as 2 days.
52. The isolated cell population according to any one of items 46 to 51, wherein the pluripotent stem cells in step c) are cultured in medium comprising DMEM/F12, B27, N2, NEAA, BME, Penicillin-Streptomycin and a TGF-β inhibitor, such as SB431542.
53. The isolated cell population according to any one of items 46 to 52, wherein the pluripotent stem cells in step c) are cultured for at least 1 days, such as 2 days.
54. The isolated cell population according to any one of items 46 to 53, wherein the pluripotent stem cells in step d) are cultured in medium comprising DMEM/F12, B27, N2, NEAA, BME, Penicillin-Streptomycin and BMP4.
55. The isolated cell population according to any one of items 46 to 54, wherein the pluripotent stem cells in step d) are cultured for at least 1 day, such as at least 2 days, such as 3 days.
56. The isolated cell population according to any one of items 46 to 55, wherein the pluripotent stem cells in step e) are cultured in medium comprising DMEM/F12, B27, N2, NEAA, BME, Penicillin-Streptomycin, bFGF and a BMP inhibitor.
57. The isolated cell population according to any one of items 46 to 56, wherein the pluripotent stem cells in step e) are cultured for at least 1 day, such as at least 2 days, such as 3 days, such as at least 5 days, such as at least 10 days, such as at least 13 days.
58. The isolated cell population according to any one of items 46 to 57, wherein the pluripotent stem cells in step f) are cultured in medium comprising DMEM/F12, B27, N2, NEAA, BME, Penicillin-Streptomycin, bFGF, IGF-1 and dbcAMP.
59. The isolated cell population according to any one of items 1 to 23, or the method according to any one of items 25 to 45, wherein the in-vitro cell population or the in-vitro mixed cell population is the in-vitro cell population of any one of items 49 to 61.
60. The method according to any one of items 25 to 59, wherein the in-vitro mixed cell population is an otic progenitors cells (OPCs) population.
61. A kit for isolating the in-vitro population comprising spiral ganglion neural progenitors (SGNPs) according to any one of items 1 to 23, said kit comprising :
   a. labelled binding members arranged to bind one or more markers from CXCR4, SUSD2, LPAR3, EPHA5, CHRNA3, REEP1, NPFFR2, SORCS3, NFASC, GABRB3, KCNB2, GRM8, DLL3 and/or KCNH8, preferably wherein said binding members are antibody(ies), nanobodies or microbodies specific for said markers, or magnetic beads conjugated to antibody(ies), nanobodies or microbodies specific for said markers;
   b. Instructions performing the method according to any one of
      items 25 to 60.
62. A vial comprising cell culture medium and an isolated cell population according to any one of items 1 to 23.
63. The isolated cell population, the method, or the vial according to any one of the preceding items, wherein the isolated cell population comprising spiral ganglion neural progenitors (SGNPs) comprises at least 1 million cells, such as at least 5 million cells, such as at least 10 million cells, such as at least 50 million cells, such as at least 100 million cells, such as at least 1 billion cells, for example at a concentration of at least 1 million cells, such as at least 5 million cells, such as at least 10 million cells, such as at least 50 million cells, such as at least 100 million cells, such as at least 1 billion cells per mL.
64. The isolated cell population, the method, or the vial according to any one of the preceding items, wherein the isolated cell population comprising spiral ganglion neural progenitors (SGNPs) comprises at least 1 million SGNPs, such as at least 5 million SGNPs, such as at least 10 million SGNPs, such as at least 50 million SGNPs, such as at least 100 million SGNPs, such as at least 1 billion SGNPs, for example at a concentration of at least 1 million SGNPs, such as at least 5 million SGNPs, such as at least 10 million SGNPs, such as at least 50 million SGNPs, such as at least 100 million SGNPs, such as at least 1 billion SGNPs per mL.

### Examples

### Example 1: Novel cells surface markers for the identification and isolation of spiral ganglion neural progenitors (SGNPs)

### Aim:

To characterize novel cells surface markers for the identification and isolation of spiral ganglion neural progenitors (SGNPs) from a heterogeneous cell population, with the goal of obtaining a pure cell population.

### Material and methods:

### In vitro Differentiation of spiral ganglion neural progenitors (SGNPs) from Human Pluripotent Stem Cells

Pluripotent stem cells (hPSCs) were initially seeded at a density of 12,000 - 24,000 cells per cm². Upon reaching 90% confluency, cells were dissociated using TrypLE (Life Technologies). The undifferentiated cells were then plated at a reduced cell density of 18,000 cells/cm². Otic induction was conducted using a methodology based on previous studies (Matsuoka et al., 2017) with minor modifications. Specifically, two days before the start of the differentiation, hPSCs were seeded at a density of 18,000 cells/cm² and cultured in stem cell medium, supplemented with 10 µM of ROCK inhibitor.

After two days, the medium was exchanged to "differentiation medium," comprising DMEM/F12, 2% B27, 1% N2, 1X NEAA, 0.1 mM BME, and 1% Penicillin-Streptomycin, supplemented with a TGF-β inhibitor for 2 days.

Afterwards, the TGF-β inhibitor was withdrawn, and BMP signaling with the addition of 10 ng/mL of BMP4 was activated for 3 days (day 5 of differentiation).

In the following days, the induction of the pre-placodal ectoderm stage was achieved by adding 100 nM of LDN-193189 (a BMP signalling inhibitor) along with 50 ng/mL of bFGF. Cells were then maintained in a "proliferative medium" comprising differentiation medium supplemented with 10 ng/mL of bFGF, 10 ng/mL of IGF-1, and 0.5 µM of dbcAMP until day 13 of differentiation.

### Single cell RNA sequencing

Cells after 13 days of differentiation were analyzed by single cell RNA-seq, following manufacturer's instructions (Document Number: CG000204, Title: Chromium Next GEM Single Cell 3' Reagent Kits v3.1 User Guide). Briefly, single cell sequencing data was pre-processed and mapped to the human genome using the cell ranger software provided by 10X and the automatically detected cells are subsequently filtered to remove cells that are most likely dead cells (low gene or UMI count, high mitochondrial gene content) or potential doublets (high gene/UMI count). Moreover, features (genes) that are detected (with a count>0) in less than 3 cells were disregarded (removed) from the downstream analysis. Due to the low per cell sequencing depth in scRNA-seq, a detection limit of 1 UMI for marker evaluations was used.

### Results:

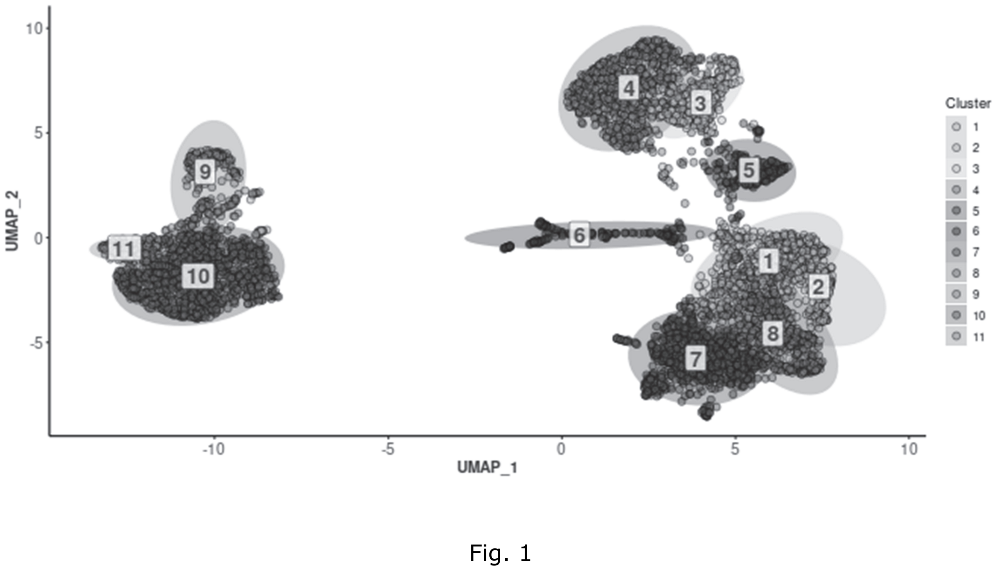

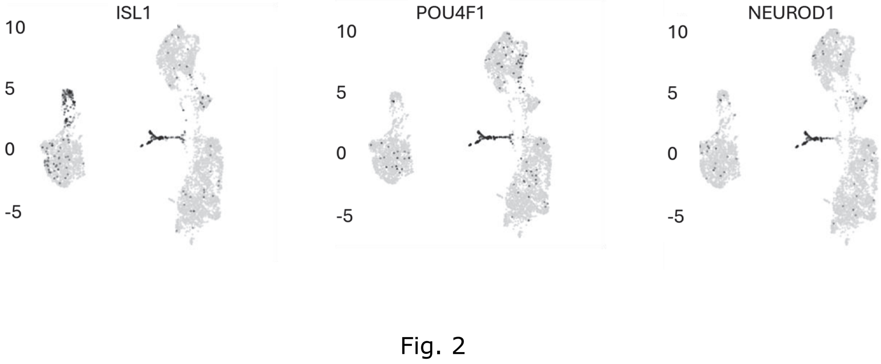

Through unbiased clustering analysis of scRNA-seq data, 11 distinct clusters were identified (Figure 1). Among these, cluster 6 was selected for further investigation due to its specific expression of key markers ISL1, POU4F1, and NEUROD1 (Figure 2), which are known as standard markers for spiral ganglion neural progenitor cells (Deng et al., 2014).

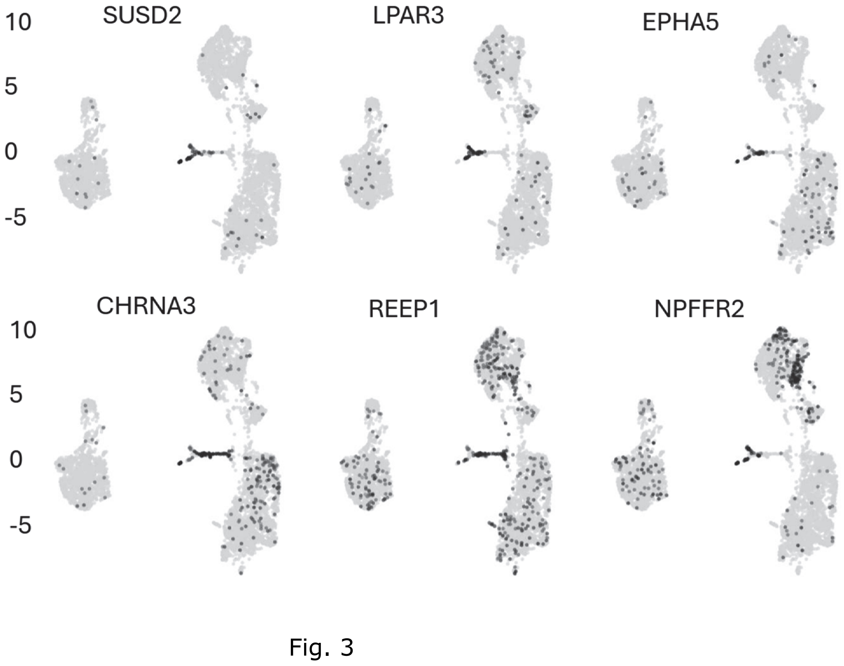

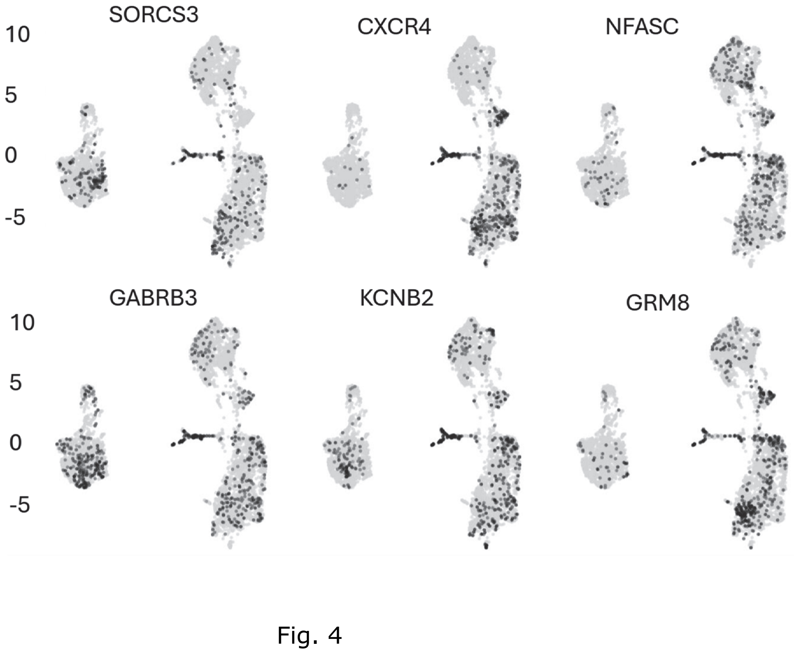

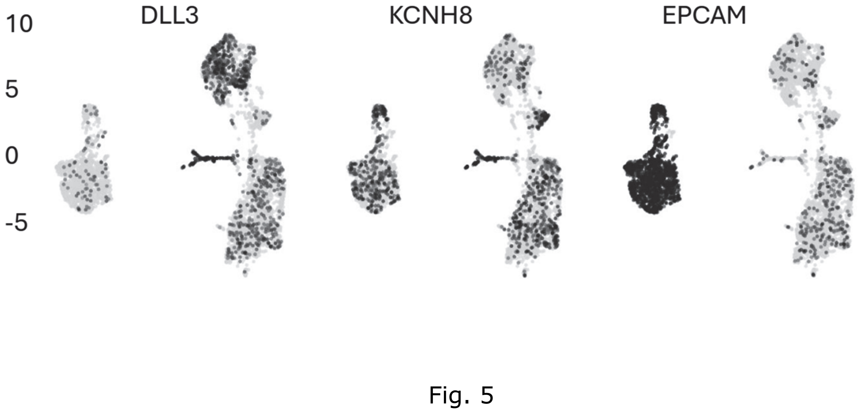

Further analysis revealed that several additional genes not normally associated with SGNPs, were enriched in cluster 6, including:
- SUSD2, LPAR3, EPHA5, CHRNA3, REEP1, and NPFFR2 (Figure 3)
- SORCS3, CXCR4, NFASC, GABRB3, KCNB2, and GRM8 (Figure 4)
- DLL3 and KCNH8 (Figure 5)

It is important to note that some markers were highly and selectively expressed in cluster 6 (such as SUSD2), while other markers were mapped onto other clusters as well (such as GABRB3). Additionally, EPCAM was identified as a potential marker to isolate or deplete clusters 9, 10, and 11 (Figure 5), making it a useful marker for negative selection of the desired SGNP population.

### Conclusion:

Using scRNA-seq clustering, the inventors of the present disclosure were able to identify a cluster of cells expressing spiral ganglion neural progenitor cells markers. The inventors were then able to identify the expression of several novel genes correlating with the spiral ganglion neural progenitor cells expression profile, within a heterogenous population of otic progenitor cells population.

### Example 2: Flow Cytometry Validation

### Aim:

To validate the surface expression of selected markers at the protein level, specifically CXCR4.

### Material and methods:

### Flow Cytometry Surface Staining for CXCR4

Following differentiation as detailed in Example 1, the proliferation medium as described in Example 1 was removed and the cells were washed twice with PBS. The cells were harvested by adding TrypLE and incubated at +37°C until detachment was observed. The cells were collected in a tube containing DMEM/F12 media and the cells were pelleted by centrifugation at 300xg for 5 minutes. Pre-cooled reagents were used. The cell pellet was resuspended in 1 mL PBS per 10⁶ cells. 1 µL of the reconstituted LIVE/DEAD^{™} dye per 1 mL of cell suspension was added. Cells were incubated at room temperature for 30 minutes, protected from light. The cells were washed by adding 1 mL Wash Buffer (PBS + 1% BSA) per 10⁶ cells. The cells were pelleted by centrifugation at 300xg for 5 min. The supernatant was aspirated, and the cell pellet was resuspended in 100 µL Wash Buffer (PBS + 1% BSA). 2 µL of CD184 (CXCR4)-APC (CXCR4-APC antibody from "CD184 (CXCR4) MicroBead Kit, human", Miltenyi Biotec, Cat. #130-100-070) was added per 10⁷ total cells. The cells were incubated at +4°C for 10 minutes. The cells were washed by adding 3 mL of Wash Buffer pelleted by centrifugation at 300xg for 5 minutes. The supernatant was aspirated and the cells resuspended in an appropriate volume of Wash Buffer. Data acquisition was performed using a flow cytometer. Compensation beads were used to account for spectral overlap between fluorochromes according to manufacturer's instructions.

### Results:

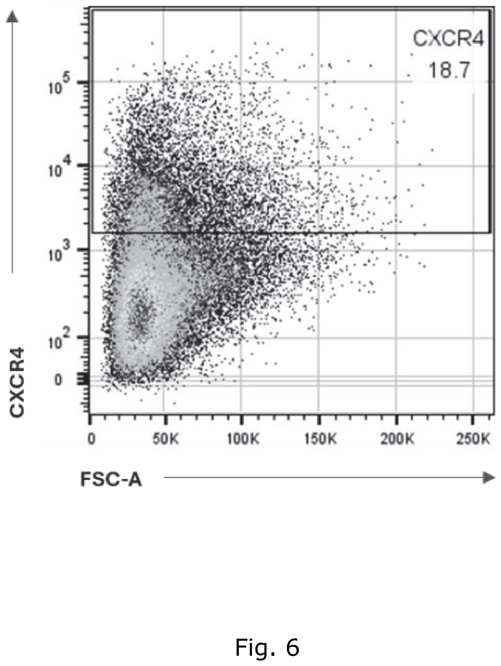

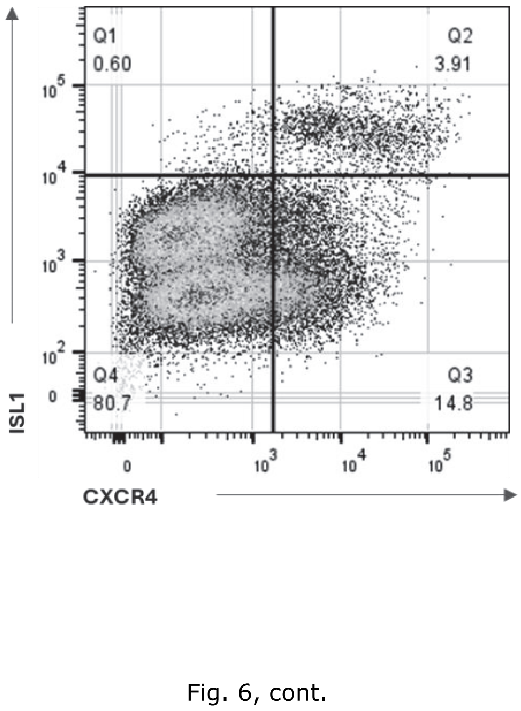

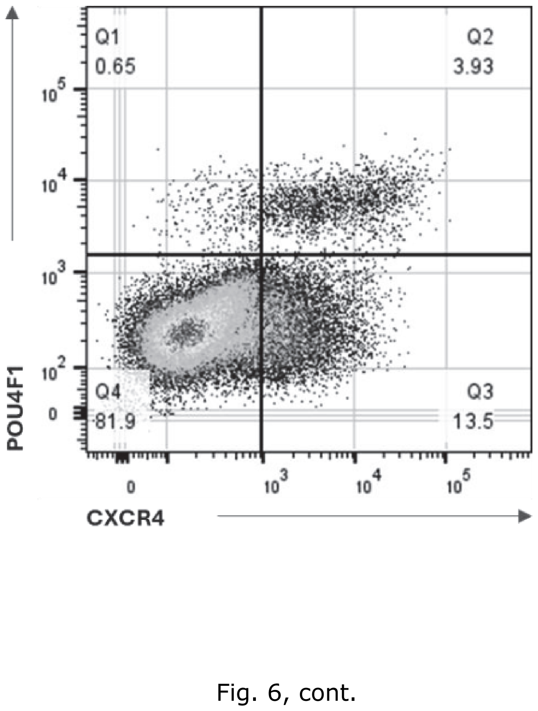

The flow cytometry results confirmed the presence of single-positive cells for CXCR4 and double-positive cells for ISL1+CXCR4+ and POU4F1+CXCR4+, supporting the scRNA-seq findings (Figure 6).

### Conclusion:

The inventors of the present disclosure conclude that CXCR4 is a key surface marker of the spiral ganglion neural progenitor cell population.

### References

M. Deng, et al. Comparative expression analysis of POU4F1, POU4F2 and ISL1 in developing mouse cochleovestibular ganglion neurons. Gene Expression Patterns 15, 31-37 (2014).
A. J. Matsuoka, et al., Directed Differentiation of Human Embryonic Stem Cells Toward Placode-Derived Spiral Ganglion-Like Sensory Neurons. Stem Cells Translational Medicine 6, 923-936 (2017).

## Claims

1. An in-vitro cell population comprising spiral ganglion neural progenitors (SGNPs), wherein said population expresses one or more markers, wherein the one or more marker is CXCR4, SUSD2, LPAR3, EPHA5, CHRNA3, REEP1, NPFFR2, SORCS3, NFASC, GABRB3, KCNB2, GRM8, DLL3, and/or KCNH8.

2. The in-vitro cell population according to any one of the preceding claims, wherein said population does not express EPCAM.

3. The in-vitro cell population according to any one of the preceding claims, wherein:
a. the marker CXCR4 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
b. the marker SUSD2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
c. the marker LPAR3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
d. the marker EPHA5 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
e. the marker CHRNA3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
f. the marker REEP1 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
g. the marker NPFFR2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
h. the marker SORCS3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
i. the marker NFASC is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
j. the marker GABRB3 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
k. the marker KCNB2 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
l. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population;
m. the marker KCNH8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population; and/or
n. the marker GRM8 is expressed in at least 1%, such as at least 2.5% such as at least 5%, such as at least 10%, such as at least 20%, such as at least 30%, such as at least 40%, such as at least 50%, such as at least 60%, such as at least 67%, such as at least 70%, such as at least 80%, such as at least 90%, such as at least 95%, such as at least 99%, such as 100% of the cells of the population.

4. The in-vitro cell population according to any one of the preceding claims, wherein said population further expresses ISL1, POU4F1, and/or NEUROD1.

5. The in-vitro cell population according to any one of the preceding claims for use in cell therapy.

6. The in-vitro cell population according to any one of the preceding claims, wherein said population comprises at least 50% SGNPs, such as at least 60% SGNPs, such as at least 75% SGNPs, such as at least 80% SGNPs, such as at least 90% SGNPs, such as at least 95% SGNPs, such as at least 99% SGNPs, such as at least 99,5% SGNPs.

7. The in-vitro cell population according to any one of the preceding claims, wherein the SGNPs have the ability to efficiently differentiate to spiral ganglion neurons (SGNs).

8. The in-vitro cell population according to claim 7, wherein at least 5%, such as at least 10%, such as at least 15%, such as at least 25%, such as at least 40%, such as at least 50%, such as at least 75%, such as at least 85%, such as at least 90%, such as at least 95% of the SGNPs differentiate to spiral ganglion neurons.

9. A method to identify spiral ganglion neural progenitors (SGNPs) from an in-vitro mixed cell population, the method comprising the step of:
a. Detecting expression of at least one marker of , CXCR4,SUSD2, LPAR3, EPHA5, CHRNA3, REEP1, NPFFR2, SORCS3 NFASC, GABRB3, KCNB2, GRM8, DLL3 and KCNH8 on single cells of the in-vitro mixed cell population;
thereby identifying a population comprising SGNPs from the in-vitro mixed population.

10. A method to isolate spiral ganglion neural progenitors (SGNPs) from an in-vitro mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
b. Isolating the cells expressing at least one of the markers;
thereby isolating a population comprising SGNPs from the in-vitro mixed population.

11. A method to enrich spiral ganglion neural progenitors (SGNPs) from an in-vitro mixed cell population, the method comprising the steps of:
a. Detecting expression of at least one marker of CXCR4, SUSD2, LPAR3, EPHA5, CHRNA3, REEP1, NPFFR2, SORCS3, NFASC, GABRB3, KCNB2, GRM8, DLL3 and KCNH8 on single cells of the in-vitro mixed cell population; and
b. Isolating the cells expressing at least one of the markers;
thereby enriching a population of SGNPs from the in-vitro mixed population.

12. The method according to any one of items 9 to 11, further comprising a step of detecting the absence of expression of a cell-surface marker, preferably EPCAM, on single cells of the in-vitro mixed cell population.

13. The method according to any one of items 9 to 12, further comprising a step of sorting from said in-vitro mixed cell population the cells in which said expression of marker(s) is detected.

14. An in-vitro cell population comprising spiral ganglion neural progenitors (SGNPs) obtained by the method comprising following steps:
a. Plating human pluripotent stem cells;
b. Culturing the pluripotent stem cells in medium;
c. Differentiating of the pluripotent stem cells in medium promoting the differentiation to otic progenitor cells;
d. Differentiating the cells of step c) in medium activating BMP signaling;
e. Culturing the cells of step d) in medium comprising a BMP signal inhibitor and bFGF; and
f. Proliferating the cells of step e) to obtain SGNPs;
g. isolating cells from the population of step f) that exhibit one or more marker is CXCR4, SUSD2, LPAR3, EPHA5, CHRNA3, REEP1, NPFFR2, SORCS3, CXCR4, NFASC, GABRB3, KCNB2, GRM8, DLL3, and/or KCNH8.

15. A kit for isolating the in-vitro population comprising spiral ganglion neural progenitors (SGNPs) according to any one of items 1 to 8, said kit comprising :
a. labelled binding members arranged to bind one or more markers from CXCR4, SUSD2, LPAR3, EPHA5, CHRNA3, REEP1, NPFFR2, SORCS3, NFASC, GABRB3, KCNB2, GRM8, DLL3 and/or KCNH8, preferably wherein said binding members are antibody(ies), nanobodies or microbodies specific for said markers, or magnetic beads conjugated to antibody(ies), nanobodies or microbodies specific for said markers;
b. Instructions performing the method according to any one of claims 9 to 13.
